(19) Europäisches Patentamt
European Patent Office
Office européen des brevets

(11) **EP 1 982 169 B1**

(12) **EUROPEAN PATENT SPECIFICATION**

(45) Date of publication and mention of the grant of the patent:
**07.11.2012 Bulletin 2012/45**

(21) Application number: **07762897.2**

(22) Date of filing: **10.01.2007**

(51) Int Cl.:
*G01N 29/024* *(2006.01)*    *G01N 29/22* *(2006.01)*
*G01N 29/34* *(2006.01)*    *G01N 29/42* *(2006.01)*
*G01F 1/66* *(2006.01)*    *G01F 1/74* *(2006.01)*
*G01N 9/24* *(2006.01)*    *G01N 33/28* *(2006.01)*

(86) International application number:
**PCT/US2007/000872**

(87) International publication number:
**WO 2007/089412 (09.08.2007 Gazette 2007/32)**

(54) **Apparatus and method for measuring parameters of a multiphase fluid flow**

Vorrichtung und Verfahren zum Messen von Parametern eines mehrphasigen Fluidflusses

Appareil et procédé de mesure des paramètres d'un écoulement de fluide polyphasique

(84) Designated Contracting States:
**AT BE BG CH CY CZ DE DK EE ES FI FR GB GR HU IE IS IT LI LT LU LV MC NL PL PT RO SE SI SK TR**

(30) Priority: **11.01.2006 US 758382 P**

(43) Date of publication of application:
**22.10.2008 Bulletin 2008/43**

(73) Proprietor: **Expro Meters, Inc.**
**Wallingford, CT 06492 (US)**

(72) Inventor: **GYSLING, Daniel**
**Glastonbury, CT 06033 (US)**

(74) Representative: **Klunker . Schmitt-Nilson . Hirsch**
**Patentanwälte**
**Destouchesstrasse 68**
**80796 München (DE)**

(56) References cited:
| | |
|---|---|
| WO-A-01/31298 | WO-A-01/69040 |
| WO-A-2005/116637 | US-A- 4 491 008 |
| US-A- 5 083 452 | US-A- 5 259 250 |
| US-A1- 2004 168 522 | US-A1- 2005 193 832 |

**Description**

**Cross-Reference To Related Patent Applications**

This application is a continuation in part of US Patent Application No. 11/442,954, filed May 30, 2006, which claimed the benefit of U.S. Provisional Application No. 60/685,532 (Cidra Docket No. CC-0818) filed May 27, 2005; and U.S. Provisional Application No. 60/736,684, (Cidra Docket No. CC-0840) filed November 14,2005; and claims the benefit of U.S. Provisional Patent Application No. 60/758,242 (Atty. Docket No. CC-0842) filed January 10, 2006.

**Technical Field**

This invention relates to an apparatus for measuring a parameter of a process flow passing within a pipe, and more particularly to a flow measurement apparatus having ultrasonic sensors and an array of strain-based sensors and for processing data signals therefrom to provide an output indicative of the speed of sound propagating through the process flow and/or a flow parameter of the process flow passing through a pipe.

**Background Art**

In industrial applications that involve flowing fluids, such as slurries, liquids, chemical, paper, pulp, petroleum, gas, pharmaceutical, food, mining, minerals and vapors and gasses in refinery, it is sometimes beneficial to know certain characteristics of the flowing fluids. For example, in the petroleum industry in which billions of dollars of crude oil are fiscally measured each day on its way from the well heads to the refineries, the volumetric flow rate is a critical measurement in process control and optimization. Unfortunately however, large amounts of hydrocarbons tend to be present in crude oil and as such, during transport between the well heads and the refineries the crude oil has a propensity to 'out gas' during transport resulting in small, unknown levels of entrained gases being present at the fiscal measurement locations. This is undesirable for at least two (2) reasons.

First, because the effect of the entrained gases on most known liquid volumetric technologies results in an over reporting of the liquid component flow rate by an amount equal to the volume of the entrained gases, the measured volumetric flow rate is typically inaccurate. In fact, standards have been imposed for volumetric flow. Unfortunately, however, while most standards for fiscal volumetric flow of liquids require that the liquid be completely devoid of gases, a problem arises when it becomes impractical to ensure that the liquid stream in question is indeed completely devoid of free gases. This is because although the gas volume fraction (GVF) level is typically less than 1%, it is often the primary source of error in the fiscal measurement. Second, because the complete separation of the gas and liquid phases cannot be ensured, the liquid volume determination is also typically inaccurate resulting in inaccurate watercut values. Thus, it is reasonable to expect that if more characteristics are known about the flowing fluid, there will be a better chance of effectively measuring, controlling, and optimizing the processing of the flowing fluid.

Accuracy of oil production measurement is limited to three constraints. One constraint involves the inability to ensure the complete separation of gas and liquid flow. This constraint results in an inaccurate liquid volume determination, inaccurate gas volume determination and an inaccurate watercut determination. The second constraint involves the relatively low number of flow measurements available due to maintenance requirements, installation requirements and pressure drop in the point with any increase in measurement points. The third constraint involves the very low number of watercut measurement points, which is due to the reliability of the watercut measurement devices and the calibration requirements of the meters.

In US 2004/0168522 A1 there is disclosed an apparatus that measures the speed of sound and/or vortical disturbances propagating in a single phase fluid flow and/or multiphase mixture to determine parameters, such as mixture quality, particle size, vapour/mass ratio, liquid/vapour ratio, mass flow rate, enthalpy and volumetric flow rate of the flow in a pipe, by measuring acoustic and/or dynamic pressures.

In US 4,491,008 there is disclosed a method of ultrasonic measurement of the ratio of the volume of gas present in an enclosure containing a liquid-gas mixture to the total volume of the enclosure. Particularly, an enclosure contains water and steam, where a portion of the steam is in the form of bubbles distributed within the water. Transducers transmit and receive high-frequency ultrasonic waves through the water and further transducers transmit and receive low-frequency ultrasonic waves through the water. A pressure detector determines the equilibrium pressure of the steam and the bubbly water inside the tank.

In WO 01/69040 A1 there is disclosed a method and apparatus for in-situ characterisation of down hole fluids in a well bore using transmitted T1 and received R1 acoustic signals. The well bore tool comprises three sets of acoustic transducers perpendicular to direction of an oil flow in the bore hole.

In US 5,083,452 A there is disclosed a method for recording multi-phase flows through a transport system in which acoustic energy signals are sensed by one of a plurality of acoustic sensors.

In US 2005/193832 A, a flowmeter includes a vibratable flowtube and a driver connected to the flowtube that is operable to impart motion to the flowtube. A sensor is connected to the flowtube and is operable to sense the motion of the flowtube and generate a sensor signal. A controller is connected to receive the sensor signal. The controller is operable to determine an individual flow rate of each phase within a multi-phase flow through the flowtube.

Thus, it would be advantageous, particularly in the oil and production field, to have a reliable, non-intrusive, clamp-on apparatus capable of measuring the parameters of an aerated multiphase fluid flow, such as the volumetric flow rate liquid of the process flow, the gas volume (or void) fraction of the flow, the watercut of the flow, and the volumetric flow rate of each of the phases of the flow. The present invention provides such an apparatus.

**Summary of the Invention**

An apparatus for determining a characteristic of an aerated fluid flowing within a pipe, according to claim 1.

The foregoing and other objects, features and advantages of the present invention will become more apparent in light of the following detailed description of exemplary embodiments thereof.

Brief Description of the Drawings

The foregoing and other features and advantages of the present invention will be more fully understood from the following detailed description of illustrative embodiments, taken in conjunction with the accompanying drawings in which like elements are numbered alike:

Figure 1 is a block diagram of a flow measurement apparatus having an array of strain-based sensors and an array of ultrasonic sensors for measuring parameters of a multiphase flow in accordance with the present invention.

Figure 2 is a plot of the measured speed of sound normalized to the speed of sound of the liquid over a frequency range in accordance with the present invention.

Figure 3 is a plot of the measured speed of sound normalized to the speed of sound of the liquid as a function of gas volume fraction in accordance with the present invention.

Figure 4 is a schematic diagram of a flow measurement apparatus of Figure 1 having an array of strain-based sensors and an array of ultrasonic sensors for measuring parameters of a multiphase flow.

Figure 5 is a cross-sectional view of a pipe having a turbulent fluid flow or mixture flowing therein, the flow having coherent structures therein, namely acoustic waves and vortical disturbances, in accordance with the present invention.

Figure 6 is a block diagram of the GVF Logic in accordance with the present invention.

Figure 7 is a block diagram of the GVF Logic in accordance with the present invention.

Figure 8 is a schematic diagram of a speed of sound (SOS) logic of an array processor of a flow measuring apparatus in accordance with the present invention.

Figure 9 is a kω plot of data processed from an apparatus embodying the present invention that illustrates the slopes of a pair of acoustic ridges, in accordance with the present invention.

Figure 10 is a plot of mixture sound speed as a function of gas volume fraction over a range of process pressures, in accordance with the present invention.

Figure 11 is a schematic diagram of a flow logic of an array processor of a flow measuring apparatus in accordance with the present invention.

Figure 12 a kω plot of data processed from an apparatus embodying the present invention that illustrates the slope of a convective ridge, and a plot of the optimization function of the convective ridge, in accordance with the present invention.

Figure 13 is a plot of the speed of sound of the liquid as a function of the volume fraction of the water in the mutiphase flow in accordance with the present invention.

Figure 14 is a block diagram of another embodiment of a flow measurement apparatus having an array of strain-based sensors and ultrasonic sensors for measuring parameters of a multiphase flow in accordance with the present invention.

Figure 15 is a schematic diagram of a flow measurement apparatus of Figure 14 having an array of strain-based sensors and an array of ultrasonic sensors for measuring parameters of a multiphase flow.

Figure 16 is a schematic diagram of a flow measurement apparatus similar to that shown in Figure 1 which includes a density and/or mass flow meter such as a coriolis meter.

Figure 17 is a clamp-on multi-phase (e.g. three phase) flow measurement apparatus comprising flow meter similar to that shown in Figure 15 having an array of strain-based sensors, a clamp-on density meter such as a nuclear densitometer, and at least one ultrasonic sensor to provide a watercut measurement, in accordance with the present invention.

Figure 18 is schematic diagram of a system for monitoring and measuring flow parameters of a fluid separator, wherein the three phase measurement device of Figure 17 is provided on the input pipe of the separator, the flow measurement device of Figure 16 is provided on the liquid leg of the separator, and the wet gas flow measurement device is provided on the gas leg of the separator.

**Detailed Description**

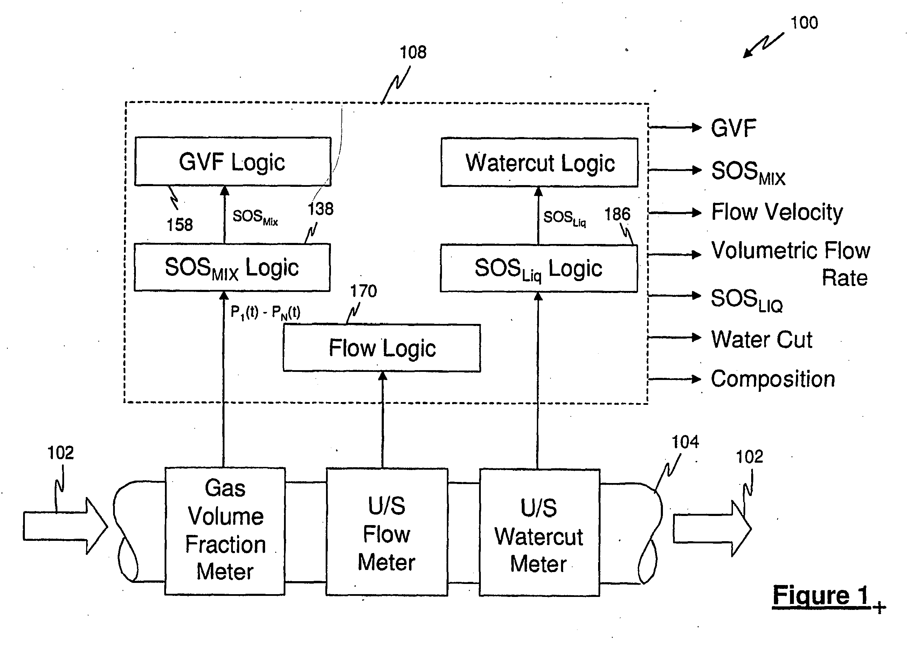

Figure 1 illustrates a block diagram of a flow measurement device 100 for measuring a parameter of a multiphase flow 102 passing through a pipe 104. The multiphase flow or mixture 102 includes any mixture having any combination of a gas, liquid, or solid phase and while the present invention is particularly useful in measuring multiphase flows, it should be appreciated that the apparatus 100 can also measure a parameter of a single phase flow. As discussed hereinbefore, the apparatus embodying the present invention is useful in measuring a multiphase flow comprising oil, water and gas. The description of the present invention will therefore assume that the mixture is a combination of oil, water, and oil, however, the invention contemplates that any single or multiphase flow can be measured.

As shown in Figure 1, the apparatus 100 functions as a gas volume fraction (or void fraction) meter, an ultrasonic flow meter, and an ultrasonic watercut meter. The gas volume fraction (GVF) meter provides an output indicative of the gas volume fraction or void fraction of the mixture 102 by measuring the speed of sound propagating at low frequencies axially through the flow 102 in the pipe 104. The ultrasonic flow meter provides a plurality of high frequency acoustic signals through the flow 102 to provide output signals indicative of pressure disturbances (e.g., vortical disturbances) propagating with the flow 102 past the ultrasonic sensors, which will be described in greater detail hereinafter. The ultrasonic watercut meter measures the speed of sound of a high frequency signal propagating through the flow 102 to provide an output signal indicative of the speed of sound of the liquid, which is indicative of the watercut of the mixture 102, wherein watercut is the phase fraction or percentage of the water in the flow 102.

It should be appreciated that the combination of the GVF meter, flow meter and watercut meter provides sufficient information to fully characterize the multiphase fluid 102 flowing through the pipe 104. Specifically, the apparatus 100 is capable of measuring at least the flow velocity, the volumetric flow rate, the flow composition (e.g., phase fraction), the watercut, the volumetric flow rate of a phase of the mixture, the gas volume (void) fraction of the flow, the speed of sound of the mixture, and the speed of sound of the liquid. One can appreciate that these measured parameters are particularly important in oil production applications.

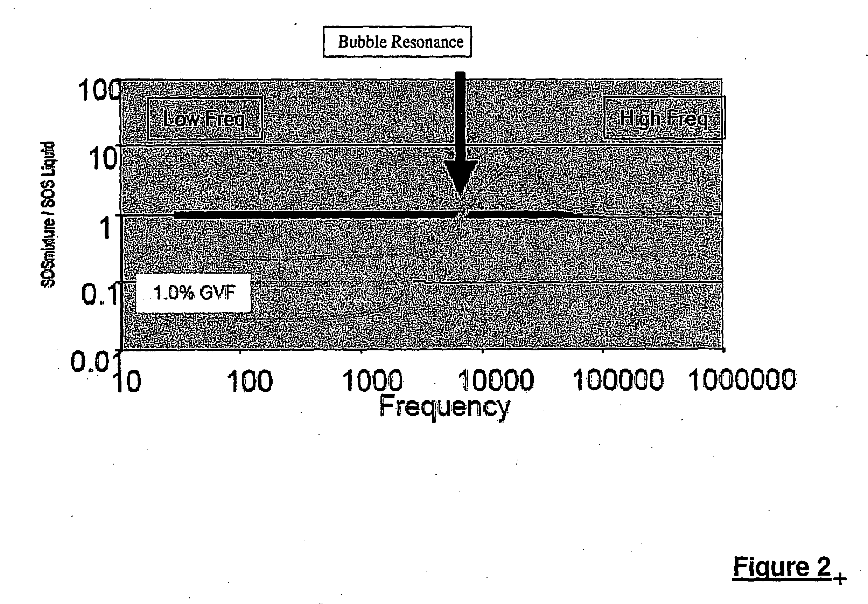

One important aspect of the present invention involves the recognition that a frequency dependence of the speed of sound propagating through the fluid flow 102 exists for bubbly fluids, wherein the bubble resonance determines the transition frequency. Figure 2 illustrates the frequency dependence of the speed of sound in bubbly fluids. As shown, at lower frequencies below the bubble resonant frequency (approximately 100 Hz to 1000 Hz), the speed of sound propagating through the fluid 102 is dramatically influenced by entrained gases. Conversely, at higher frequencies above the bubble resonant frequency (approximately 1 MHz and greater), entrained gas in the fluid flow 102 has no significant impact on the speed of sound propagating through the liquid. Recognizing this phenomenon, the apparatus 100 embodying the present invention provides a meter, such as a GVF meter, to measure the speed of sound at low frequencies below the bubble resonant frequency, and another meter, such as an ultrasonic watercut meter, to measure the speed of sound at high frequencies above the bubble resonant frequency.

As will be described in greater detail hereinafter, the measured speed of sound at the lower frequency (e.g., sub-resonant frequencies) is indicative of the speed of sound of the mixture 102, while the measured speed of sound at the higher frequencies (e.g., super-resonant frequencies) is indicative of the speed of sound of the liquid. Knowing the speed of sound of the mixture 102 enables the gas volume (and void) fraction of the flow 102 (or mixture) to be determined. Further, knowing the speed of sound of the liquid enables the watercut to be determined. This processing will be described in greater detail hereinafter.

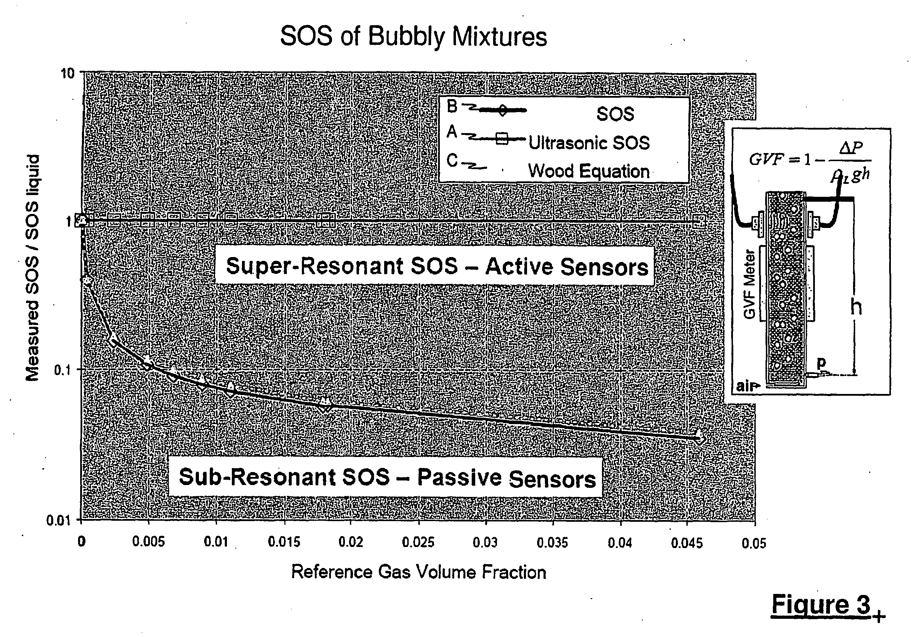

Tests were performed using a vertical pipe filled with a fluid, wherein bubbles were injected into the fluid at the bottom of the pipe. Using an ultrasonic sensor and a GVF meter, the speed of sound at super-resonant frequencies and sub-resonant frequencies, respectively, were measured. Referring to Figure 3, the data obtained illustrates the phenomenon described hereinbefore that the measured speed of sound of the liquid (e.g., super-resonant SOS) is not affected by the entrained gas, while the measured speed of sound of the mixture 102 (e.g., sub-resonant SOS) is affected by the entrained gas. Additionally, the data in Figure 3, which represents the illustrates the effects of the speed of sound of bubble mixtures or flows 102. Specifically, the measured speed of sound normalized by the liquid speed of sound is plotted as a function of the reference gas volume fraction.

The line A in Figure 3 shows the normalized measured super-resonant speed of sound as a function of the referenced GVF. As discussed hereinbefore, the measured speed of sound at higher frequencies (super-resonant) is not affected by entrained gas and is indicative of the speed of sound of the liquid regardless of the amount of entrained gas.

The line B in Figure 3 shows the normalized measured sub-resonant speed of sound as a function of the referenced GVF. As discussed hereinbefore, the measured sound speed at lower frequencies (sub-resonant) is affected by entrained gas by a known or determinable relationship, thus enabling the determination of the gas volume (or void) fraction of the multiphase flow or mixture 102.

The line C in Figure 3 shows the theoretical normalized sub-resonant speed of sound of the mixture 102 as a function of the referenced GVF in accordance with the Woods equation.. As can be seen, the measured sub-resonant speed of sound correlates with the theoretical determination of the sub-resonant speed of sound.

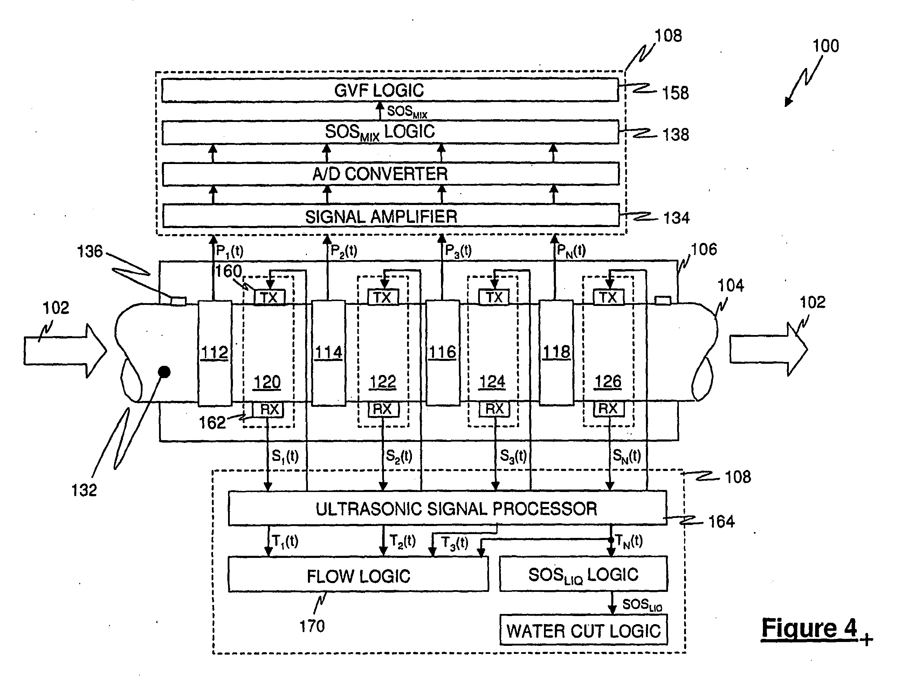

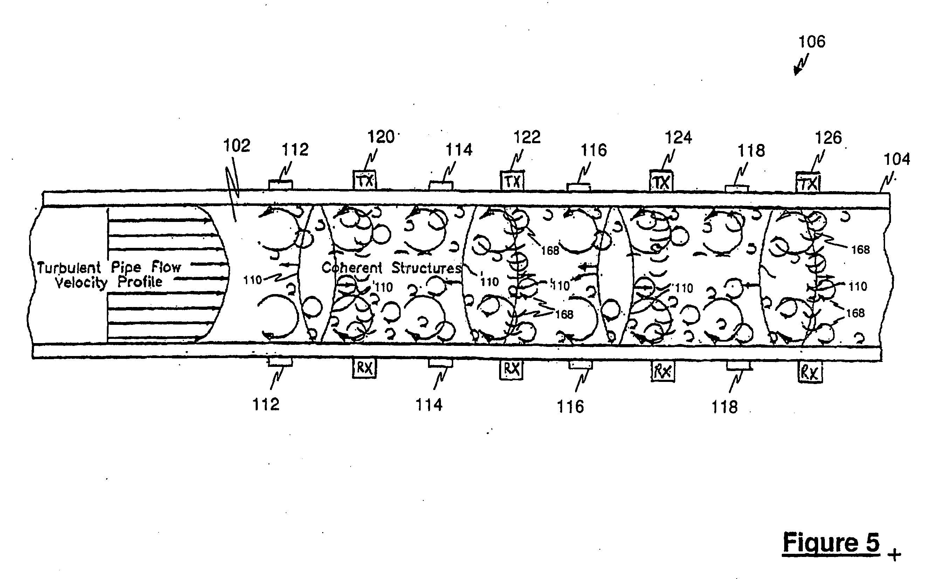

Referring to Figure 4 a schematic diagram of the flow measurement apparatus 100 of Figure 1 is illustrated, wherein the flow measurement apparatus 100 includes a sensing device (sensor head) 106 mounted to a pipe 104 and a processing unit or array processor (transmitter) 108. In accordance with the present invention, the apparatus 100 can determine the speed at which sound (i.e., acoustic wave 110 in Figure 5) propagates through the fluid flow 102 within the pipe 104 to measure particular characteristics of the single or multi-phase fluids. To simplify the explanation of the present invention, the flow 102 propagating through the pipe 104 will be referred to as a process flow with the understanding that the fluid or process flow 102 may be a single phase or multi-phase flow, as described hereinbefore.

The sensing device 106 comprises an array of strain-based sensors or pressure sensors 112-118 for measuring the unsteady pressures produced by acoustic pressure disturbances (e.g., acoustic waves 110) within the pipe 104 to determine the speed of sound propagating through the flow 102. The sensing device 106 further includes an array of ultrasonic sensors 120-126, each of which have a transmitter 160 and a receiver 162 to also measure a parameter of the flow 102. Although the pressure sensors 112-118 and ultrasonic sensors 120-126 are shown interlaced, it should be appreciated that each respective sensor array may be partially interlaced or not interlaced at all without departing from the present invention. It is also contemplated that the GVF meter and the ultrasonic flow meter may be two distinct units disposed adjacent to each other on the pipe 104.

The pressure signals $P_1(t)$ - $P_N(t)$ generated by the pressure sensors 112-118 and the ultrasonic signals $S_1(t)$ - $S_N(t)$ generated by the ultrasonic sensors 120-126 are provided to the processing unit 108, which digitizes the signals and computes the appropriate flow parameter(s). A cable electronically connects the sensing device 106 to the processing unit 108. The analog pressure sensor signals $P_1(t)$ - $P_N(t)$ are typically 4-20 mA current loop signals.

The array of pressure sensors 112-118 comprises an array of at least two pressure sensors 118, 120 spaced axially along the outer surface 132 of the pipe 104, having a process flow 102 propagating therein. The pressure sensors 112-118 may be clamped onto or generally removably mounted to the pipe 104 by any releasable fastener, such as bolts, screws and clamps. Alternatively, the sensors 112-118 may be permanently attached to or integral (e.g., embedded) with the pipe 104. It should be appreciated that the array of sensors 112-118 of the sensing device 106 may include any number of pressure sensors 18-21 greater than two sensors, such as three, four, eight, sixteen or N number of sensors between two and twenty-four sensors. Generally, the accuracy of the measurement improves as the number of sensors in the array increases, wherein the degree of accuracy provided by the greater number of sensors is typically offset by the increase in complexity and time for computing the desired output parameter of the flow 102. Therefore, the number of sensors used is dependent at least in part on the degree of accuracy desired and the desire update rate of the output parameter provided by the apparatus 100. The pressure sensors 112-118 measure the unsteady pressures produced by acoustic waves propagating through the flow 102 within the pipe 104, which are indicative of the SOS propagating through the fluid flow 102 in the pipe 104. The output signals ($P_1(t)$- $P_N(t)$) of the pressure sensors 112-118 are provided to a signal amplifier 134 that amplifies the signals generated by the pressure sensors 112-118. The processing unit 108 processes the pressure measurement data $P_1(t)$-$P_N(t)$ and determines the desired parameters and characteristics of the flow 102, as described hereinbefore.

The apparatus 100 also contemplates providing one or more acoustic sources 136 to enable the measurement of the speed of sound propagating through the flow 102 for instances of acoustically quiet flow. The acoustic source 136 may be a device that taps or vibrates on the wall of the pipe 104, for example. The acoustic sources 136 may be disposed at the input end or the output end of the array of sensors 112-118, or at both ends as shown. One should appreciate that in most instances the acoustic sources 136 are not necessary and the apparatus 100 passively detects the acoustic ridge provided in the flow 102, as will be described in greater detail hereinafter. The passive noise includes noise generated by pumps, valves, motors, and the turbulent mixture itself.

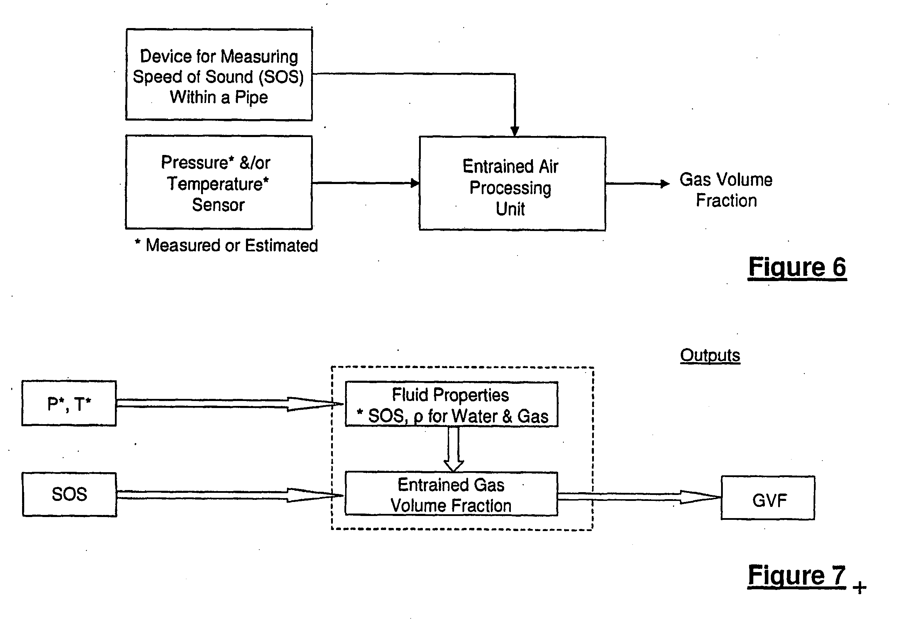

Generally, the processing unit 108 measures unsteady pressures created by acoustical disturbances propagating through the flow 102 to determine the speed of sound (SOS) propagating through the flow 102. Knowing the pressure and/or temperature of the flow 102 and the speed of sound of the acoustic disturbances or waves, as shown in Figure 6 and Figure 7, the processing unit 108 can determine the volumetric flow of the fluid, the consistency or composition of the fluid, the Mach number of the fluid, the average size of particles flowing through the fluid, the air/mass ratio of the fluid, and/or the percentage of entrained air within the mixture 102, such as that described in U.S. Patent Application No. 10/349,716 (CiDRA Docket No. CC-0579), filed January 23, 2003, U.S. Patent Application No. 10/376,427 (CiDRA Docket No. CC-0596), filed February 26, 2003, U.S. Patent Application No. 10/762,410 (CiDRA Docket No. CC-0703), filed January 21,2004.

As shown in Figure 4, an apparatus 100 embodying the present invention has an array of at least two strain-based or pressure sensors 112-114, located at two locations $x_1$, $x_2$ axially along the pipe 104 for sensing respective stochastic signals propagating between the sensors 112-114 within the pipe 104 at their respective locations. Each sensor 112-114 provides a signal indicating an unsteady pressure at the location of each sensor, at each instant in a series of sampling instants. One should appreciate that the sensor array may include more than two pressure sensors as depicted by pressure sensors 116, 118 at location $x_3$, $x_N$. The pressure generated by the acoustic waves 110 (see Figure 5) may be measured through strained-based sensors and/or pressure sensors 112-118. The pressure sensors 112-118 provide analog pressure time-varying signals $P_1(t), P_2(t), P_3(t), P_N(t)$ to the signal processing unit 108.

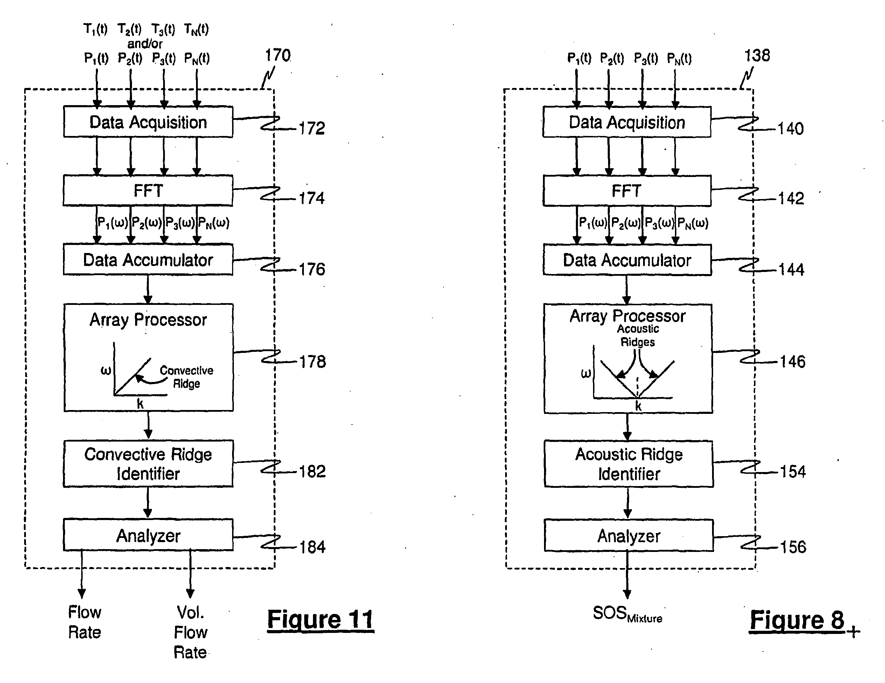

As shown in Figure 8, the SOS Mixture Logic 138 includes a data acquisition unit 140 that digitizes the pressure signals $P_1(t)$-$P_N(t)$ associated with the acoustic waves 110 propagating through the pipe 104. An FFT logic 142 calculates the Fourier transform of the digitized time-based input signals $P_1(t)$ - $P_N(t)$ and provide complex frequency domain (or frequency based) signals $P_1(\omega), P_2(\omega), P_3(\omega), P_N(\omega)$ indicative of the frequency content of the input signals.

A data accumulator 144 accumulates the signals $P_1(t)$ - $P_N(t)$ from the sensors, and provides the data accumulated over a sampling interval to an array processor 146, which performs a spatial-temporal (two-dimensional) transform of the sensor data, from the xt domain to the k-$\omega$ domain, and then calculates the power in the k-$\omega$ plane, as represented by a k-$\omega$ plot, similar to that provided by the convective array processor 178 discussed further hereinafter.

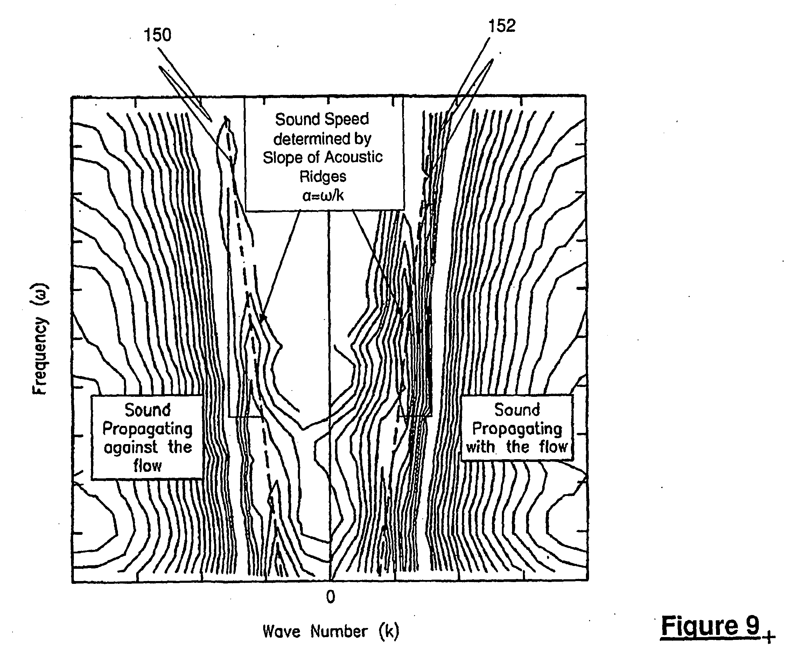

To calculate the power in the k-$\omega$ plane, as represented by a k-$\omega$ plot (see Figure 9) of either the signals or the differenced signals, the array processor 146 determines the wavelength and so the (spatial) wavenumber k, and also the (temporal) frequency and so the angular frequency $\omega$, of various of the spectral components of the stochastic parameter. There are numerous algorithms available in the public domain to perform the spatial/temporal decomposition of the array of pressure sensors 112-118.

Specifically, the array processor 146 uses standard so-called beam forming, array processing, or adaptive array-processing algorithms, i.e. algorithms for processing the sensor signals using various delays and weighting to create suitable phase relationships between the signals provided by the different sensors, thereby creating phased antenna array functionality. In other words, the beam forming or array processing algorithms transform the time domain signals from the sensor array into their spatial and temporal frequency components, i.e. into a set of wave numbers given by k=2$\pi$/$\lambda$ where $\lambda$ is the wavelength of a spectral component, and corresponding angular frequencies given by $\omega$=2$\pi\nu$.

One such technique of determining the speed of sound propagating through the flow 102 involves using array processing techniques to define an acoustic ridge in the k-$\omega$ plane as shown in Figure 9. The slope of the acoustic ridge is indicative of the speed of sound propagating through the flow 102. The speed of sound (SOS) is determined by applying sonar arraying processing techniques to determine the speed at which the one dimensional acoustic waves 110 propagate past the axial array of unsteady pressure measurements distributed along the pipe 104.

The apparatus 100 of the present invention measures the speed of sound (SOS) of one-dimensional sound waves 110 (see Figure 5) propagating through the mixture 102 to determine the gas volume fraction of the mixture 102. It is known that sound propagates through various mediums at various speeds in such fields as SONAR and RADAR fields. The speed of sound propagating through the pipe 104 and flow 102 may be determined using a number of known techniques, such as those set forth in U.S. Patent Application Serial No. 09/344,094, filed June 25, 1999, now US 6,354,147; U.S. Patent Application Serial No. 10/795,111, filed March 4, 2004; U.S. Patent Application Serial No. 09/997,221, filed November 28, 2001, now US 6,587,798; U.S. Patent Application Serial No. 10/007,749, filed November 7, 200 1, and U.S. Patent Application Serial No. 10/762,410, filed January 21, 2004.

In the case of suitable acoustic waves 110 being present in both axial directions, the power in the k-$\omega$ plane shown in a k-$\omega$ plot of Figure 9 so determined will exhibit a structure that is called an acoustic ridge 150, 152 in both the left and right planes of the plot, wherein one of the acoustic ridges 150 is indicative of the speed of sound traveling in one axial direction and the other acoustic ridge 152 being indicative of the speed of sound traveling in the other axial direction.

The acoustic ridges 150, 152 represent the concentration of a stochastic parameter that propagates through the flow 102 and is a mathematical manifestation of the relationship between the spatial variations and temporal variations described above. Such a plot will indicate a tendency for k-$\omega$ pairs to appear more or less along a line 150, 152 with some slope, the slope indicating the speed of sound. The power in the k-$\omega$ plane so determined is then provided to an acoustic ridge identifier 154, which uses one or another feature extraction method to determine the location and orientation (slope) of any acoustic ridge 150, 152 present in the left and right k-$\omega$ plane. An analyzer 156 determines the speed of sound of the mixture 102 by using the slope of one of the two acoustic ridges 150, 152 or averaging the slopes of the acoustic ridges 150, 152.

As shown in Figure 1 and Figure 4, the GVF logic 158 provides output signals indicative of gas volume or void fraction of the mixture 102 in response to the measured speed of sound of the mixture 102. For example, to determine the gas volume fraction (or phase fraction), the GVF logic 158 assumes a nearly isothermal condition for the flow 102. As such the gas volume fraction or the void fraction is related to the speed of sound by the following quadratic equation:

$$Ax^2 + Bx + C = 0$$

wherein x is the speed of sound, A=1+rg/r1*($K_{eff}$/P-1)-$K_{eff}$/P, B=$K_{eff}$/-2+rg/r1; C=1-$K_{eff}$/r1*$a_{meas}$^2); Rg = gas density, r1 = liquid density, $K_{eff}$ = effective K (modulus of the liquid and pipewall), P= pressure, and $a_{meas}$ = measured speed of sound.

Effectively,

**Gas Volume Fraction (GVF) = (-B+sqrt(B^2-4\*A\*C))/(2\*A)**

Alternatively, the sound speed of a mixture can be related to volumetric phase fraction ($\phi_i$) of the components and the sound speed (a) and densities (p) of the component through the Wood equation.

$$\frac{1}{\rho_{mix} a^2_{mix\infty}} = \sum_{i=1}^{N} \frac{\phi_i}{\rho_i a_i^2} \qquad \text{where} \qquad \rho_{mix} = \sum_{i=1}^{N} \rho_i \phi_i$$

One dimensional compression waves propagating within a mixture 102 contained within a pipe 104 exerts an unsteady internal pressure loading on the pipe 104. The degree to which the pipe 104 displaces as a result of the unsteady pressure loading influences the speed of propagation of the compression wave. The relationship among the infinite domain speed of sound and density of a mixture, the elastic modulus (E), thickness (t), and radius (R) of a vacuum-backed cylindrical conduit, and the effective propagation velocity ($a_{eff}$) for one dimensional compression is given by the following expression:

$$a_{eff} = \frac{1}{\sqrt{\dfrac{1}{a^2_{mix\infty}} + \rho_{mix} \dfrac{2R}{Et}}} \qquad \text{(eq 1)}$$

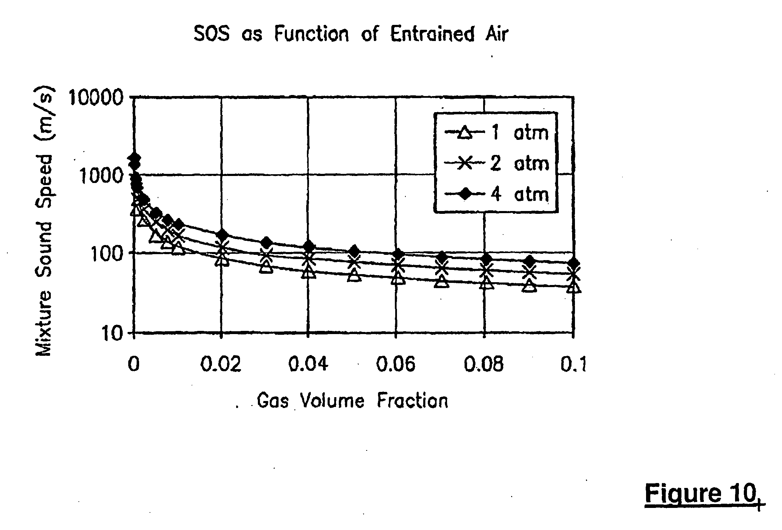

The mixing rule essentially states that the compressibility of a mixture ($1/(\rho a^2)$) is the volumetrically-weighted average of the compressibilities of the components. For gas/liquid mixtures 102 at pressure and temperatures typical of the paper and pulp industry, the compressibility of gas phase is orders of magnitudes greater than that of the liquid. Thus, the compressibility of the gas phase and the density of the liquid phase primarily determine mixture sound speed, and as such, it is necessary to have a good estimate of process pressure to interpret mixture sound speed in terms of volumetric fraction of entrained gas. The effect of process pressure on the relationship between sound speed and entrained air volume fraction is shown in Figure 10.

Some or all of the functions within the processing unit 108 may be implemented in software (using a microprocessor or computer) and/or firmware, or may be implemented using analog and/or digital hardware, having sufficient memory, interfaces, and capacity to perform the functions described herein.

As shown in Figure 4, the measurement apparatus 100 includes a sensing device 106 comprising an array of ultrasonic sensor units 120-126. Each sensor unit 120-126 comprises a pair of ultrasonic sensors 160, 162, one of which functions as a transmitter (Tx) 160 and the other as a receiver (Rx) 162. The sensor units 120-126 are spaced axially along the outer surface 132 of the pipe 104 having a process flow 102 propagating therein. The pair of sensors 160, 162 is diametrically disposed on the pipe 104 at predetermined locations along the pipe 104 to provide a through transmission configuration, such that the sensors transmit and receive an ultrasonic signal that propagates through the fluid substantially orthogonal to the direction of the flow of the fluid within the pipe 104. The ultrasonic measurement portion of the present invention is similar to that shown in U.S. Provisional Patent Application No. 10/756,977 (Atty Docket No. CC-0700) filed on January 13, 2004.

As shown in Figure 1, each pair of ultrasonic sensors 160, 162 measures a transit time (i.e., time of flight (TOF), or phase modulation) of an ultrasonic signal propagating through the fluid 102 from the transmitting sensor 160 to the receiving sensor 162. The transit time measurement or variation is indicative of coherent properties that convect with the flow within the pipe 104 (e.g., vortical disturbances, inhomogenieties within the flow, temperature variations, bubbles, particles, pressure disturbances), which are indicative of the velocity of the process flow 102. The ultrasonic sensors 160, 162 may operate at any frequency, however, it has be found that the higher frequency sensors are more suitable for single phase fluids while lower frequency sensors are more suitable for multiphase fluids. The optimum frequency of the ultrasonic sensors 160, 162 is dependent on the size or type of particle or substance propagating with the flow 102. For instance, the larger the air bubbles in an aerated fluid the lower the desirable frequency of the ultrasonic signal. Examples of frequency used for a flow meter embodying the present invention are 1 MHz and 5 MHz. The ultrasonic sensors 160,162 may also provide a pulsed, chirped or continuous signal through the fluid flow 102. An example of the sensors 160, 162 that may be used are Model no. 113-241-591, manufactured by Krautkramer.

An ultrasonic signal processor 164 fires the sensors 160, 162 in response to a firing signal from the transmitter 108 and receives the ultrasonic output signals $S_1(t)$ - $S_N(t)$ from the sensors 160, 162. The signal processor 164 processes the data from each of the sensor units 120-126 to provide an analog or digital output signal $T_1(t)$ - $T_N(t)$ indicative of the time of flight or transit time of the ultrasonic signal through the fluid. The signal processor 164 may also provide an output signal indicative of the amplitude (or attenuation) of the ultrasonic signals. One such signal processor is model no. USPC 2100 manufactured by Krautkramer Ultrasonic Systems. Measuring the amplitude of ultrasonic signals is particularly useful and works best for measuring the velocity of a fluid that includes a substance in the flow (e.g., multiphase fluid or slurry).

The output signals ($T_1(t)$- $T_N(t)$) of the ultrasonic signal processor 164 are provided to the processor 108, which processes the transit time or modulation measurement data to determine the volumetric flow rate. The transit time or time of flight measurement is defined by the time it takes for an ultrasonic signal to propagate from the transmitting sensor 160 to the respective receiving sensor 162 through the pipe wall and the fluid 102. The effect of the vortical disturbances (and/or other inhomogenities within the fluid) on the transit time of the ultrasonic signal is to delay or speed up the transit time. Therefore, each sensing unit 120-126 provides a respective output signal $T_1(t)$-$T_N(t)$ indicative of the variations in the transit time of the ultrasonic signals propagating orthogonal to the direction of the fluid 102. The measurement is derived by interpreting the convecting coherent property and/or characteristic within the process piping using at least two sensor units 120, 122. The ultrasonic sensors 120-126 may be "wetted" or clamped onto the outer surface 132 of the pipe 104 (e.g. contact or non-contact sensor).

In one example, the flow meter 100 measures the volumetric flow rate by determining the velocity of vortical disturbances or "eddies" 168 (see Figure 5) propagating with the flow 102 using the array of ultrasonic sensors 120-126. The flow meter 100 measures the velocities associated with unsteady flow fields created by vortical disturbances or "eddies" 168 and other inhomogenities to determine the velocity of the flow 102. The ultrasonic sensor units 120-126 measure the transmit time $T_1(t)$-$T_N(t)$ of the respective ultrasonic signals between each respective pair of sensors 160, 162, which vary due to the vortical disturbances as these disturbances convect within the flow 102 through the pipe 104 in a known manner. Therefore, the velocity of these vortical disturbances is related to the velocity of the flow 102 and hence the volumetric flow rate may be determined, as will be described in greater detail hereinafter. The volumetric flow is determined by multiplying the velocity of the fluid by the cross-sectional area of the pipe 104.

The Flow Logic 170 of the processing unit 108 processes the ultrasonic signals as shown in Figure 11, wherein the Flow Logic 170 receives the ultrasonic signals from the array of sensors 120-126. A data acquisition unit 172 (e.g., A/D converter) converts the analog signals to respective digital signals and the digitized signals are provided to Fast Fourier Transform (FFT) logic 174. The FFT logic 174 calculates the Fourier transform of the digitized time-based input signals $T_1(t)$ - $T_N(t)$ and provides complex frequency domain (or frequency based) signals $T_1(\omega)$, $T_2(\omega)$, $T_3(\omega)$, $T_N(\omega)$ indicative of the frequency content of the input signals. It should be appreciated that instead of FFT's, any other technique for obtaining the frequency domain characteristics of the signals $T_1(t)$ - $T_N(t)$, may be used. For example, the cross-spectral density and the power spectral density may be used to form a frequency domain transfer functions (or frequency response or ratios) discussed hereinafter.

One technique of determining the convection velocity of the turbulent eddies 168 within the process flow 102 (see Figure 5) is by characterizing a convective ridge of the resulting unsteady pressures using an array of sensors or other beam forming techniques, similar to that described in U.S Patent Application, Serial No. (Cidra's Docket No. CC-0122A) and U.S. Patent Application, Serial No. 09/729,994 (Cidra's Docket No. CC-0297), filed December 4, 200, now US6,609,069.

A data accumulator 176 accumulates the frequency signals $T_1(\omega)$ - $T_N(\omega)$ over a sampling interval, and provides the data to an array processor 178, which performs a spatial-temporal (two-dimensional) transform of the sensor data, from the xt domain to the k-$\omega$ domain, and then calculates the power in the k-$\omega$ plane, as represented by a k-$\omega$ plot.

The array processor 178 uses standard so-called beam forming, array processing, or adaptive array-processing algorithms, i.e. algorithms for processing the sensor signals using various delays and weighting to create suitable phase relationships between the signals provided by the different sensors, thereby creating phased antenna array functionality. In other words, the beam forming or array processing algorithms transform the time domain signals from the sensor array into their spatial and temporal frequency components, i.e. into a set of wave numbers given by k=$2\pi/\lambda$ where $\lambda$ is the wavelength of a spectral component, and corresponding angular frequencies given by $\omega=2\pi\nu$.

The prior art teaches many algorithms of use in spatially and temporally decomposing a signal from a phased array of sensors, and the present invention is not restricted to any particular algorithm. One particular adaptive array processing algorithm is the Capon method/algorithm. While the Capon method is described as one method, the present invention contemplates the use of other adaptive array processing algorithms, such as MUSIC algorithm. The present invention recognizes that such techniques can be used to determine flow rate, i.e. that the signals caused by a stochastic parameter convecting with a flow are time stationary and have a coherence length long enough that it is practical to locate sensor units apart from each other and yet still be within the coherence length.

Convective characteristics or parameters have a dispersion relationship that can be approximated by the straight-line equation,

$$k=\omega/u,$$

where u is the convection velocity (flow velocity). A plot of k-$\omega$ pairs obtained from a spectral analysis of sensor samples associated with convective parameters portrayed so that the energy of the disturbance spectrally corresponding to pairings that might be described as a substantially straight ridge, a ridge that in turbulent boundary layer theory is called a convective ridge. What is being sensed are not discrete events of turbulent eddies 168, but rather a continuum of possibly overlapping events forming a temporally stationary, essentially white process over the frequency range of interest. In other words, the convective eddies 168 is distributed over a range of length scales and hence temporal frequencies.

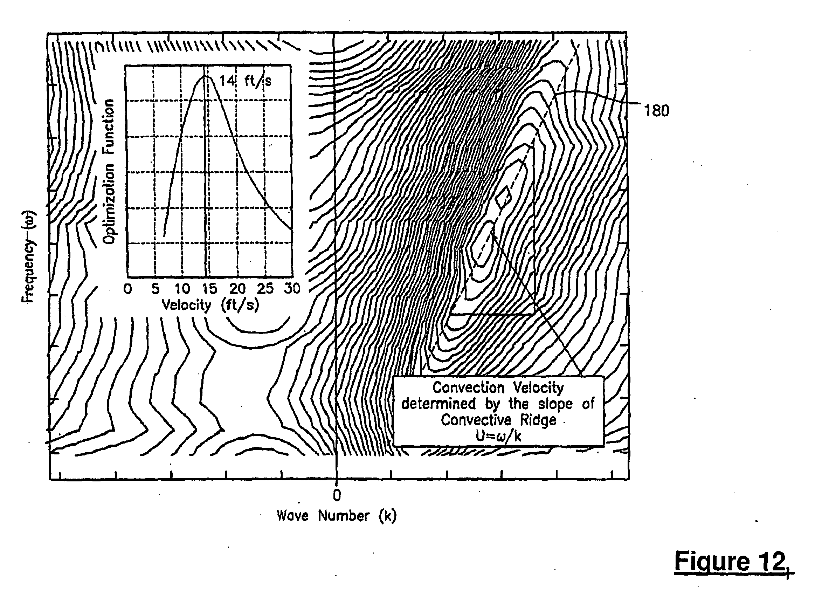

To calculate the power in the k-$\omega$ plane, as represented by a k-$\omega$ plot (see Figure 12) of either the signals, the array processor 178 determines the wavelength and so the (spatial) wavenumber k, and also the (temporal) frequency and so the angular frequency $\omega$, of various of the spectral components of the stochastic parameter. There are numerous algorithms available in the public domain to perform the spatial/temporal decomposition of arrays of sensor units 120-126.

The present invention may use temporal and spatial filtering to precondition the signals to effectively filter out the common mode characteristics $P_{common\ mode}$ and other long wavelength (compared to the sensor spacing) characteristics in the pipe 104 by differencing adjacent sensors and retain a substantial portion of the stochastic parameter associated with the flow field and any other short wavelength (compared to the sensor spacing) low frequency stochastic parameters.

In the case of suitable turbulent eddies 168 (see Figure 5) being present, the power in the k-$\omega$ plane shown in a k-$\omega$ plot of Figure 12 shows a convective ridge 180. The convective ridge 180 represents the concentration of a stochastic parameter that convects with the flow 102 and is a mathematical manifestation of the relationship between the spatial variations and temporal variations described above. Such a plot will indicate a tendency for k-$\omega$ pairs to appear more or less along a line 180 with some slope, the slope indicating the flow velocity.

Once the power in the k-$\omega$ plane is determined, a convective ridge identifier 182 uses one or another feature extraction method to determine the location and orientation (slope) of any convective ridge 180 present in the k-$\omega$ plane. In one embodiment, a so-called slant stacking method is used, a method in which the accumulated frequency of k-$\omega$ pairs in the k-$\omega$ plot along different rays emanating from the origin are compared, each different ray being associated with a different trial convection velocity (in that the slope of a ray is assumed to be the flow velocity or correlated to the flow velocity in a known way). The convective ridge identifier 182 provides information about the different trial convection velocities, information referred to generally as convective ridge information.

The analyzer 184 examines the convective ridge information including the convective ridge orientation (slope). Assuming the straight-line dispersion relation given by k=$\omega$/u, the analyzer 184 determines the flow velocity, Mach number and/or volumetric flow. The volumetric flow is determined by multiplying the cross-sectional area of the inside of the pipe 104 with the velocity of the process flow 102.

The watercut of the process flow 102 is determined using the output of at least one of the sensors 120-126 of the ultrasonic flow meter. While an ultrasonic sensor 120 of the ultrasonic meter is used to determine the watercut of the flow 102, it is contemplated that a separate ultrasonic sensor may be used to determine watercut. A separate ultrasonic sensor for measuring watercut would allow the sensor to transmit an ultrasonic signal at different frequencies to ensure the ultrasonic sensor for watercut is operating at a frequency greater than the bubble resonant frequency.

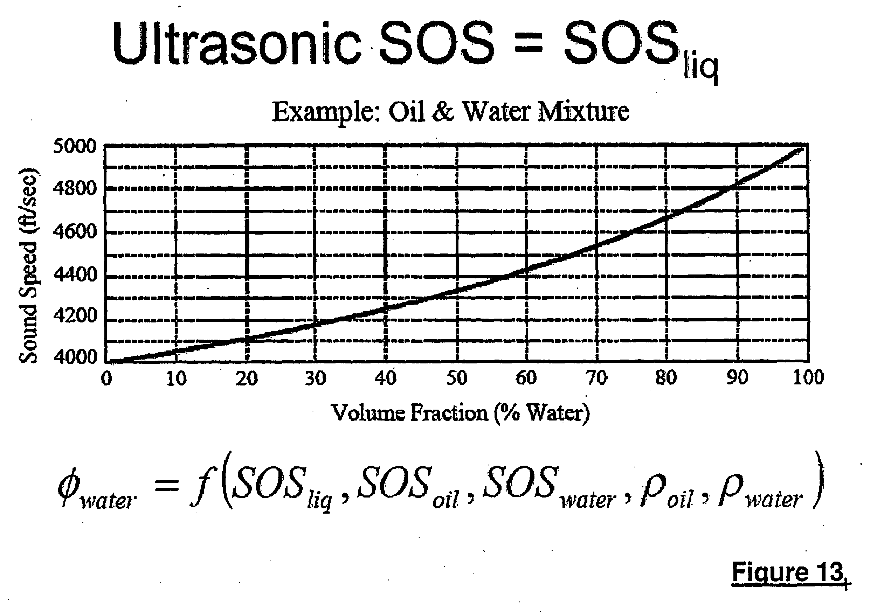

The SOS Liquid Logic 186 converts the measured transit time of the ultrasonic signal to a signal indicative of the speed of sound of the liquid. The frequency of the ultrasonic signal propagating through the fluid is greater than the bubble resonant frequency such that the entrained gas does not affect the ultrasonic signal. Knowing the SOS of the liquid portion of the fluid flow 102, the phase fraction of the water can be determined. The phase fraction of the water is a function of the SOS of the liquid, the SOS of the oil, SOS of the water, the density of the oil, and the density of the water. Knowing the SOS and density of the oil and water, the relationship between the phase fraction (e.g., watercut) of the flow 102 and the SOS of the liquid is known. As shown in Figure 13, this relationship is illustrated in the plot of SOS of the liquid v. watercut, and therefore, knowing the SOS of the liquid, the watercut may be determined.

While the sonar-based flow meter using an array of sensors to measure the speed of sound of an acoustic wave propagating through the mixture 102 is shown and described, one will appreciate that any means for measuring the speed of sound of the acoustic wave may used to determine the entrained gas volume fraction of the mixture/fluid or other characteristics of the flow 102 described hereinbefore.

While data acquisition units 140, 172, FFT logic 142, 174, data accumulators 144, 176, array processors 146, 178 and ridge identifiers 154, 182 are shown as separate elements or separate software/processing routines, one will appreciate that each of these elements may be common and able to process the data associated with both the pressure signals associated with the speed of sound and the pressures that convect with the process flow.

While each of the ultrasonic sensor units 120-126 of Figure 1 comprises a pair of ultrasonic sensors (transmitter and receiver) 160, 162 diametrically-opposed to provide through transmission, the present invention contemplates that one of the ultrasonic sensors 160, 162 of each sensor unit 120-126 may be offset axially such that the ultrasonic signal from the transmitter sensor 160 has an axial component in its propagation direction.

The present invention also contemplates the sensor units 120-126 of the sensing device 106 may be configured in a pulse/echo configuration. In this embodiment, each sensing unit 120-126 comprises one ultrasonic sensor that transmits an ultrasonic signal through the pipe wall and fluid substantially orthogonal to the direction of flow and receives a reflection of the ultrasonic signal reflected back from the wall of the pipe to the ultrasonic sensor.

The sensing device 106 may be configured to function in a pitch and catch configuration. In this embodiment, each sensor unit 120-126 comprises a pair of ultrasonic sensors (transmitter, receiver) 160, 162 disposed axially along the pipe 104 disposed on the same side of the pipe 104 at a predetermined distance apart. Each transmitter sensor 160 provides an ultrasonic signal at a predetermined angle into the flow 102. The ultrasonic signal propagates through the fluid 102 and reflects off the inner surface of the pipe 104 and reflects the ultrasonic signal back through the fluid to the respective receiver sensor 162.

As shown in Figure 1, while the ultrasonic sensor portion of the flow measurement device 100 comprises an array of ultrasonic sensor units 120-126 (see Figure 5), the present invention contemplates that any ultrasonic meter or sensing portion may be used. The ultrasonic meter may be any meter within any of the three classes of flow meters that utilize ultrasonic transducers, which include transit time ultrasonic flow meters (TTUF), doppler ultrasonic flow meters (DUF), and cross correlation ultrasonic flow meters (CCUF).

The ultrasonic sensor portion may be any known ultra-sonic flow meter, such as U.S. Patent No. 2,874,568; U.S. Patent No. 4,004,461; U.S. Patent No. 6,532,827; U.S. Patent No. 4,195,517; U.S. Patent No. 5,856,622; and U.S. Patent No. 6,397,683.

It should be appreciated that the array-based flow meter 100 is similar to that described in U.S Patent Application, Serial No. 10/007,749 filed November 7,2001 (Atty. Docket No. CC-0066B), U.S Patent Application, Serial No. 10/007,736 filed November 8, 2001 (Atty. Docket No. CC-0122A), U.S. Patent No. 6,587,798, filed on November 28,2001, (Atty. Docket No. CC-0295), U.S. Provisional Patent Application, Serial No. 60/359,785 filed February 26, 2002 (Atty. Docket No. CC-0403), U.S Provisional Patent Application, Serial No. 60/425,436 filed November 12, 2002 (Atty. Docket No. CC-0538), U.S. Patent Application Serial No. 09/729,994, filed December 4, 2000 (Atty. Docket No. 297), and U.S. Patent Application, Serial No. 10,875,857 (Atty. Docket No. CC-0749) filed June 24, 2004.

While a single array processor 108 is shown to receive and process input signals from the pressure sensors 112-118 and the ultrasonic sensors 120-126, the present invention contemplates that an array processor may be dedicated to each of the array of pressure sensors 112-118 and the array of ultra-sonic sensors 120-126.

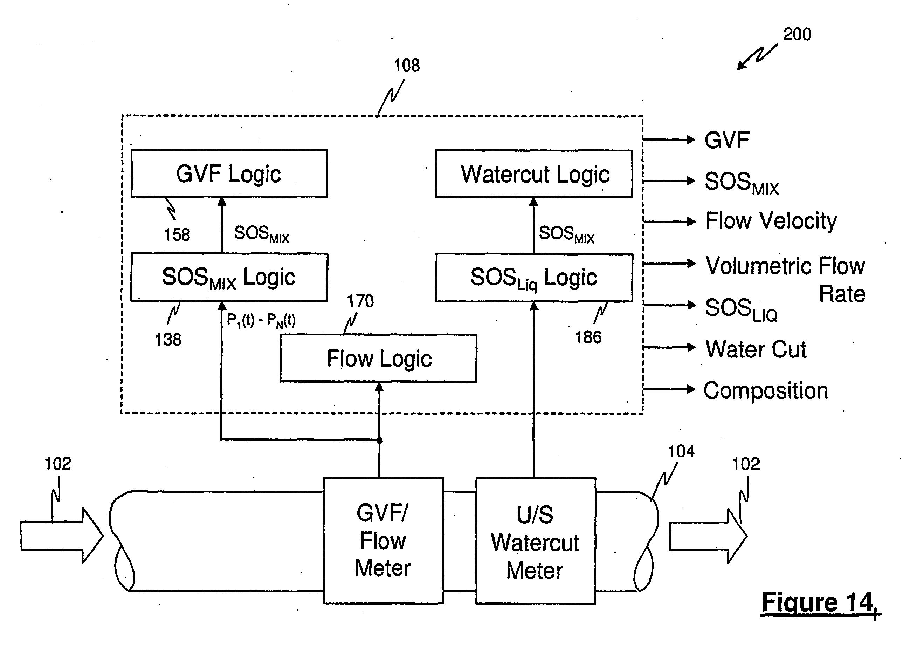

Figure 14 illustrates a block diagram of a flow measurement apparatus 200 similar to the apparatus 100 of Figure 1 that includes a sensing device (sensor head) 106 mounted to a pipe 104 and a processing unit or array processor (transmitter) 108, wherein the apparatus 200 functions as a GVF meter, a flow meter, and a watercut meter. In this embodiment, the sensor head 106 for the GVF meter functions as the sensor head 106 for both the GVF meter and flow meter of Figure 1. It should be appreciated that the processing of all the data is similar to that described hereinbefore and like reference numbers are the same elements and function the same as that described herein before.

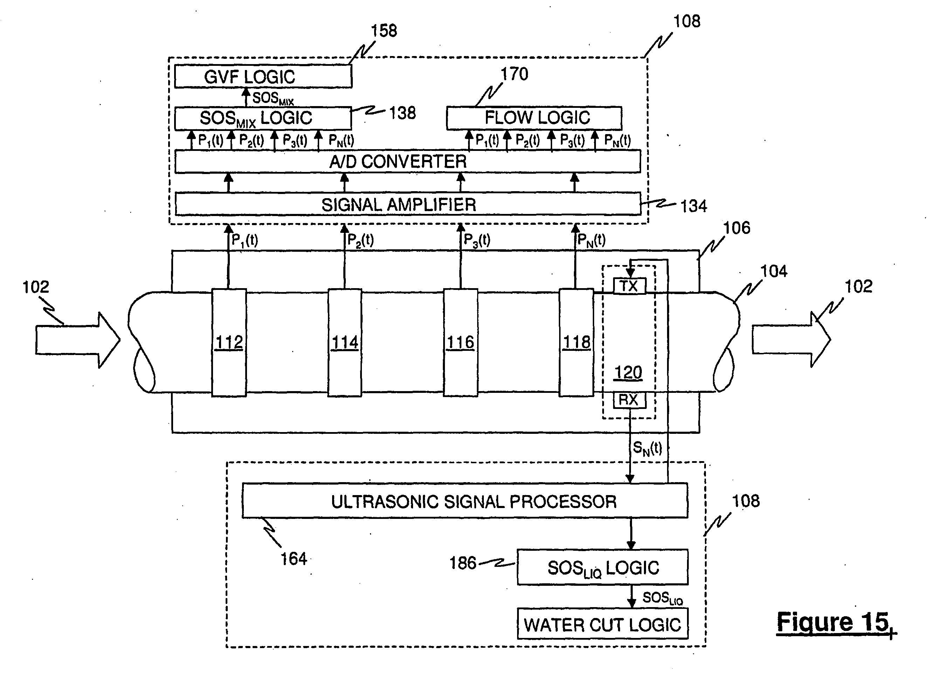

Referring to Figure 15, the sensor head 106 includes an array of strained-based or pressure sensors 112-118. The signals provided by the pressure sensors 112-118 are processed to determine the gas volume (or void) fraction of the flow 102, the velocity of the flow 102, the volumetric flow rate, and speed of sound of the mixture (i.e., flow) 102. The combination GVF/flow meter, in accordance with the present invention, can determine the speed at which sound (i.e., acoustic wave 110 in Figure 5) propagates through the fluid flow 102 within a pipe 104 to measure the speed of sound of the mixture 102 and the gas void (or volume) fraction of the flow 102. The GVF/flow meter also determines the speed at which pressure disturbances (e.g., vortical disturbances) propagate through the pipe 104 to determine the velocity of the fluid flow 102. The pressure disturbances may be in the form of vortical disturbances 168 (e.g., turbulent eddies 168 in Figure 5) or other pressure disturbances that convect (or propagate) with the flow 102.

As suggested and further described in greater detail hereinafter, the apparatus 100, 200 has the ability to measure the speed of sound (SOS) and flow rate (or velocity) using one or both of the following techniques using the same array of pressure sensors described herein below:

1) Determining the speed of sound of acoustical disturbances or sound waves propagating through the flow 102 using the array of pressure sensors 112-118, and/or
2) Determining the velocity of pressure disturbances (e.g., vortical eddies 168) propagating through the flow 102 using the array of pressure sensors 112-118.

These techniques are similar to what was taught and described hereinbefore in reference to Figure 8 and Figure 11, respectively. Also, the processing relating to the watercut meter is similar to that described herein before.

One skilled in the art should appreciate that the watercut meter may also be used as a stand alone meter to enable a user to measure the watercut of a multiphase fluid flow having entrained air.

The pressure sensors 112-118 and the ultrasonic sensors 120-126 shown in the apparatus 100, 200 in Figure 4 and Figure 15, respectively, may be clamp-on, non-wetted sensors. These clamp-on sensors allow the apparatus 100, 200 to be retro fitted onto pipes without having to shut down the system. The apparatus 100, 200 also would not interfere with the fluid flow and not create any back pressure of the fluid flow. Another advantage of the non-wetted, clamped on sensors is that corrosion or scaling does not interfere with the sensors.

In one embodiment as shown in Figure 4 and Figure 15, each of the pressure sensors 112-118 may include a piezoelectric film attached to a unitary multi-band strap to measure the unsteady pressures of the flow 102 using either technique described hereinbefore. The piezoelectric film sensors 112-118 may be mounted onto a unitary substrate or web which is mounted or clamped onto the outer surface 132 of the pipe 104, which will described in greater detail hereinafter.

The piezoelectric film sensors 112-118 include a piezoelectric material or film 188 to generate an electrical signal proportional to the degree that the material is mechanically deformed or stressed. The piezoelectric sensing element 188 is typically conformed to allow complete or nearly complete circumferential measurement of induced strain to provide a circumferential-averaged pressure signal. The sensors can be formed from PVDF films, co-polymer films, or flexible PZT sensors, similar to that described in "Piezo Film Sensors Technical Manual" provided by Measurement Specialties, Inc., which is incorporated herein by reference. A piezoelectric film sensor that may be used for the present invention is part number 1-1002405-0, LDT4-028K, manufactured by Measurement Specialties, Inc. While the piezoelectric film material is provided substantially the length of the band, and therefore the circumference of the pipe 104, the present invention contemplates that the piezoelectric film material may be disposed along a portion of the band of any length less than the circumference of the pipe 104.

Piezoelectric film ("piezofilm"), like piezoelectric material, is a dynamic material that develops an electrical charge proportional to a change in mechanical stress. Consequently, the piezoelectric material measures the strain induced within the pipe 104 due to unsteady or stochastic pressure variations (e.g., vortical and/or acoustical) within the process flow 102. Strain within the pipe 104 is transduced to an output voltage or current by the attached piezoelectric sensor 112-118. The piezoelectrical material or film may be formed of a polymer, such as polarized fluoropolymer, polyvinylidene fluoride (PVDF). The piezoelectric film sensors are similar to that described in U.S. Patent Application Serial No. 10/712,818 (CiDRA Docket No. CC-0675), filed November 12, 2003 and U.S. Patent Application Serial No. 10/795,111 (CiDRA Docket No. CC-0731), filed March 4,2004. The advantages of this clamp-on technique using piezoelectric film include non-intrusive flow rate measurements, low cost and measurement techniques that require no excitation source. One should appreciate that the sensor may be installed or mounted to the pipe 104 as individual sensors or all the sensors mounted as a single unit as shown in Figure 4 and Figure 15.

The pressure sensors 112-118 of Figure 4 described herein may be any type of sensor, capable of measuring the unsteady (or ac or dynamic) pressures or parameter that convects with the flow within a pipe 104, such as piezoelectric, optical, capacitive, resistive (e.g., Wheatstone bridge), accelerometers (or geophones), velocity measuring devices, displacement measuring devices, ultra-sonic devices, etc. If optical pressure sensors are used, the sensors 112-118 may be Bragg grating based pressure sensors, such as that described in US Patent Application, Serial No. 08/925,598, entitled " High Sensitivity Fiber Optic Pressure Sensor For Use In Harsh Environment", filed Sept. 8, 1997, now U.S. Patent 6,016,702, and in US Patent Application, Serial No. 10/224,821, entitled " Non-Intrusive Fiber Optic Pressure Sensor for Measuring Unsteady Pressures within a Pipe". In an embodiment of the present invention that utilizes fiber optics as the pressure sensors 112-118 they may be connected individually or may be multiplexed along one or more optical fibers using wavelength division multiplexing (WDM), time division multiplexing (TDM), or any other optical multiplexing techniques.

In certain embodiments of the present invention, a piezo-electronic pressure transducer may be used as one or more of the pressure sensors 112-118 and it may measure the unsteady (or dynamic or ac) pressure variations inside the pipe 104 by measuring the pressure levels inside of the pipe 104. These sensors may be ported within the pipe to make direct contact with the process flow 102. In an embodiment of the present invention, the sensors comprise pressure sensors manufactured by PCB Piezotronics. In one pressure sensor there are integrated circuit piezoelectric voltage mode-type sensors that feature built-in microelectronic amplifiers, and convert the high-impedance charge into a low-impedance voltage output. Specifically, a Model 106B manufactured by PCB Piezotronics is used which is a high sensitivity, acceleration compensated integrated circuit piezoelectric quartz pressure sensor suitable for measuring low pressure acoustic phenomena in hydraulic and pneumatic systems.

It is also within the scope of the present invention that any strain sensing technique may be used to measure the variations in strain in the pipe 104, such as highly sensitive piezoelectric, electronic or electric, strain gages and piezo-resistive strain gages attached to the pipe 104. Other strain gages include resistive foil type gages having a race track configuration similar to that disclosed U.S. Patient Application Serial No. 09/344,094, filed June 25, 1999, now US 6,354,147. The invention also contemplates strain gages being disposed about a predetermined portion of the circumference of pipe 104. The axial placement of and separation distance $\Delta X_1$, $\Delta X_2$ between the strain sensors are determined as described hereinabove.

It is also within the scope of the present invention that any other strain sensing technique may be used to measure the variations in strain in the pipe 104, such as highly sensitive piezoelectric, electronic or electric, strain gages attached to or embedded in the pipe 104.

While the description has described the apparatus as a single meter that measure the GVF, Flow and watercut, each function may be separated into individual meters for measuring GVF, flow and watercut.

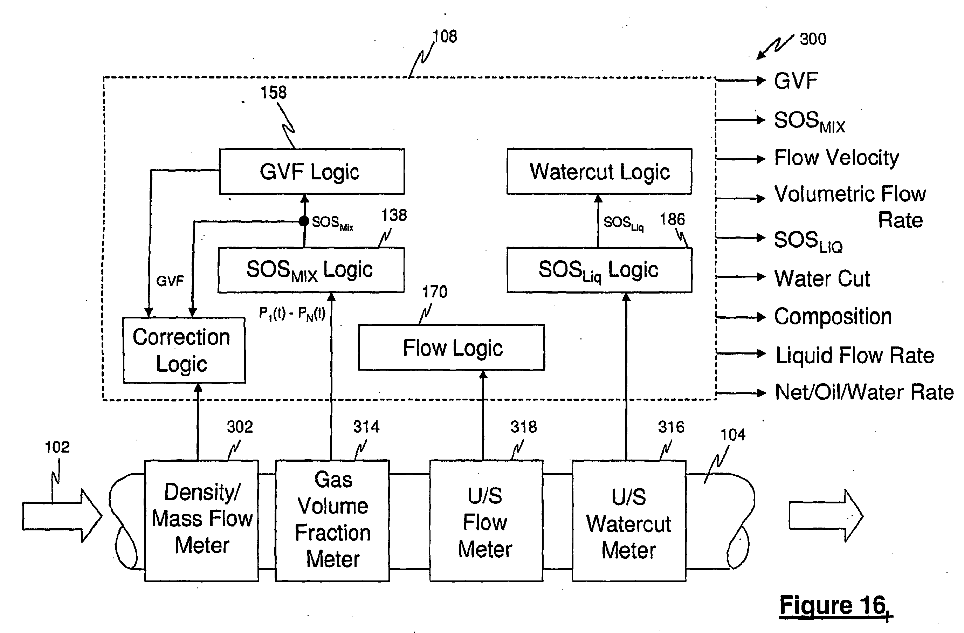

Referring to Figure 16, the description and function of the gas volume fraction meter, the ultrasonic flow meter and the ultrasonic watercut meter of the flow apparatus 300 is similar to that described hereinbefore. The flow apparatus includes a density and/or mass flow meter 302, such as a coriolis meter, to provide measurements of different parameters of the fluid flow 102. For example, the combination of the coriolis meter and the gas volume fraction meter may be an augmented output measurement of the density, mass flow, net oil flow rate, and net water flow rate (for a flow comprising an aerated oil/water mixture). This combination is similar to that described in US Patent Application No. 10/892,886 (Atty. Docket No. CC-0762) filed July 15, 2004.

For example, one approach at correcting inaccuracies in densitometers involves integrating a speed-of-sound measurement of the process fluid with the natural frequency measurement of a vibrating tube density meter to form a system with an enhanced ability to operate accurately in aerated fluids. Introducing a real time, speed-of-sound measurement address the effects of aeration on multiple levels with the intent to enable vibrating-tube-based density measurement to continue to report liquid density in the presence of entrained air with accuracy approaching that for a non-aerated liquid. Firstly, by measuring the process sound speed with process pressure, the aeration level of the process fluid can be determined with high accuracy on a real time basis. Secondly, the real time measurements of sound speed, and the derived measurement of gas volume fraction, are then utilized with empirically derived correction factors to improve the interpretation of the measured natural frequency of the vibrating tubes in terms of the density of the aerated fluid. Thirdly, the combined knowledge of aerated mixture density and aerated mixture sound speed, enable the determination of the non-aerated liquid component density, providing improved compositional information. Note liquids phase typically includes pure liquids, mixtures of liquids, as well as liquid / solid mixtures.

For densitometers, such as a Corilois meter, a decrease in the accuracy of the measurments with the introduction of bubbly fluids is well documented. In fact, others have attempted to correct for the effect of entrained air by correlating observed terrors in mass flow to the gas volume fraction within the process fluid. These authors proposed a correction based on GVF as follows:

$$R = \frac{2\alpha}{1-\alpha}$$

where $\alpha$ represents the gas volume fraction and R represents decrease in measured (apparent) mass flow normalized by the true mass flow. Thus, using this correlation, a 1% increase in entrained air would result in a roughly 2% underestimate of the actual mass flow. Although this formulation appears to capture the general trend observed experimentally, it has two drawbacks for use in the field. Firstly, the Coriolis meter typically has no direct way to measure the gas volume fraction. It has been suggested to use the measured apparent density of the fluid to estimate the level of entrained air, however, this is problematic since both of the two fundamental measurements, phase difference and natural frequency, are impacted by changes in the reduced frequency of the Coriolis vibration. Secondly, it is unlikely that the gas volume fraction is the only variable influencing the relationship between measured phase difference and mass flow and the measured natural frequency and density. Although gas volume fraction appears to correlate over at least some range of parameters, the physics of the problem suggest that sound speed, via a reduced frequency effect, may also have a direct influence on the data interpretation.

One method would be to use a direct sound measurement from the process fluid to aid in the interpretation of the Coriolis meter. In this interpretation, the reduced frequency parameters developed herein is included in interpreting the relation between the phase difference in the vibrating tubes and the mass flow as well as a direct role in interpreting the natural frequency of the oscillating flow tubes in terms of process fluid density. The sound speed measurement, combined with knowledge of process liquid and gas components as well as process temperature and pressure, enables a direct measurement of entrained air as well. Thus, the reduced frequency parameter and gas volume fraction can be used as inputs in the interpretation of phase lag in terms of mass flow. Due to the strong relationship between air content in liquids and mixture sound speed, the role of the reduced frequency parameter in the interpretation of the fundamental measurement of the Coriolis meter will have a more pronounce effect in bubbly flows. However, changes in sound speed and hence reduced frequency of operation in various types of liquids and other process mixtures have an effect on the interpretation and hence accuracy of Coriolis meter used in these applications as well. Consider, for example, the performance of a Coriolis meter on two liquids - water and oil. Assuming that the fluids have different densities and sound speeds, the different fluid properties suggest that the Coriolis meter will be operating at different reduced frequencies. The reduced frequency for the water will typically be ~10%-30% lower than that for the oil application. Recognizing that, while they are different, the reduced frequencies for both applications are still "small", the impact on accuracy may not be significant. However, some degree of inaccuracy is introduced by not accounting for the differences in the reduced frequency of operation of the Coriolis meter in this application. For other density meter, such as a nuclear densitometer, these meters may corrected simply knowing the gas volume fraction (or gas void fraction) of the fluid .

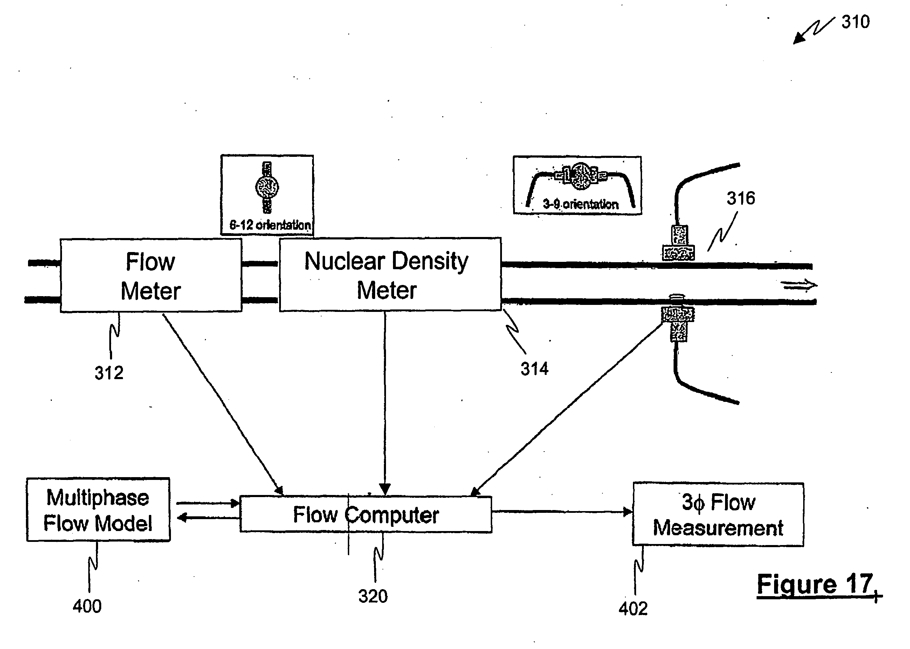

Referring to Figure 17, a clamp-on three phase flow measurement apparatus 310 is shown that provides a phase fraction measurement of the fluid flow and a volumetric flow rate of each of the phases of the flow 102. The flow may be full or partially full (i.e., stratified). The clamp on apparatus 310 comprises a flow meter 312 having a plurality of strained-based sensors disposed longitudinally along the pipe 104 similar to that shown in Figure 15. The flow meter 312 processes the data from the array of sensors similar to that described hereinbefore as indicated in the flow logic of the processing unit 108 of Figure 15 and Figure 12, to provide a fluid flow velocity. The clamp-on apparatus 310 further includes a clamp on density meter 314, such as a nuclear densitometer, wherein the sensors of the densitometer are positioned or oriented at approximately 6 and 12 o'clock or top and bottom of the pipe 104 to ensure the radiant beam pass through both gas and liquid of a stratified flow. The densitometer provides a density measurement, which is used to determine the gas volume fraction of the 3-phase fluid. The clamp-on apparatus 310 further includes at least one ultrasonic sensor 316 for determining the watercut of the liquid phase of the three phase fluid 102. The sensor 316 is disposed orthogonal to the sensors of the densitometer at 3 and 9 o'clock or in the horizontal position to ensure the ultrasonic beam or signal propagates primarily through the liquid of a stratified fluid flow. The data and/or sensed signals of the three clamped on devices 312, 314, 316 are provided to a flow computer 320 which processes the data using a multiphase flow model 400 to provide three phase flow measurements 402 of the fluid flow 102, such as compositional data (e.g., phase fraction of each phase of the fluid), velocity of each phase of the fluid 102, volumetric flow rate of each phase, and mass flow rate of each phase. It should be appreciated that the multiphase flow model 400 receives the flow data from each device 312, 314 and 316 and processes the flow data to optimize and correct for any errors, inaccuracies, and/or various flow conditions or regimes. This optimized output flow data is then output as three phase flow measurement data 402.

Referring again to Figure 17, although the sensors of the densitometer 314 (and thus the beam direction) are shown as being disposed vertically in a six o'clock and twelve o'clock position relative to the flow 102, it should be appreciated that the sensors (and thus beam) of the densitometer 314 may be disposed in any orientation relative to the flow 102 suitable to the desired end purpose. For stratified flows, a gamma densitometer may be sensitive to stratification when the beam is traversing the fluid flow in the vertical direction. In effect the densitometer measures the height of an interface rather than the holdup or gas volume fraction. By rotating the densitometer a small amount, the sensitivity of the densitometer to the stratification or partially filled pipe is reduced. Rotating the beam off the vertical axis by approximately 26.5 degrees has shown an improvement in the measurement.

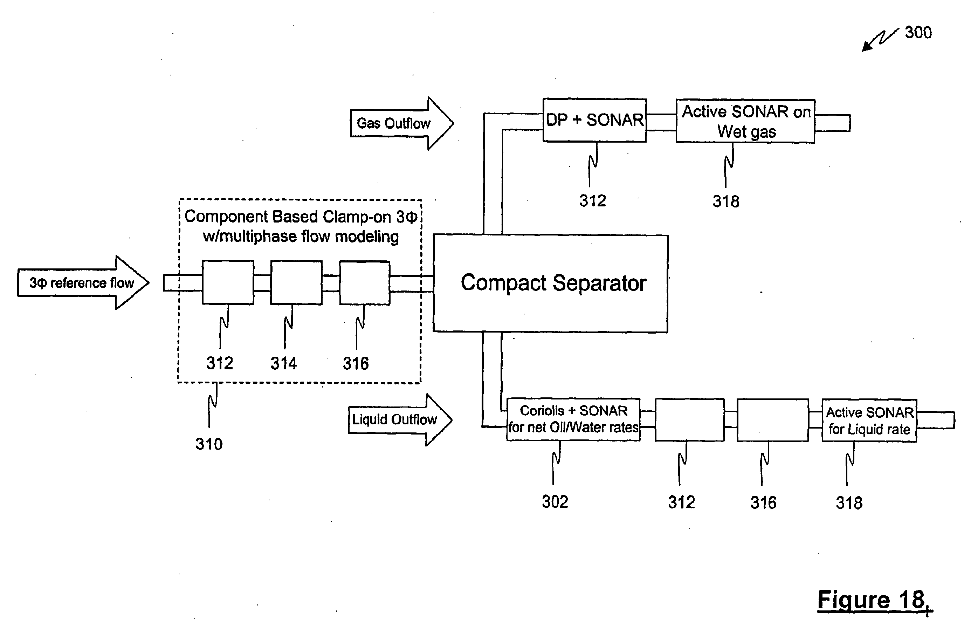

Figure 18 illustrates a schematic diagram of well surveillance system, wherein the input and output flows of a fluid separator is provided. Specifically, the clamp-on multiphase measurement apparatus 310 of Figure 17 is used to measure the flow passing within the input pipe of the separator. The measurement apparatus of Figure 16 is provided on the liquid leg of the separator for measuring the parameters of the aerated liquid mixture (e.g., aerated oil and water mixture). The gas leg of the separator includes a wet-gas flow measurement device similar to that described in US Provisional Patent Application No. 60/724,952 (Atty. Docket No. CC-0832) filed October 6, 2005; and US Provisional Patent Application No. 60/697,479 (Atty. Docket No. CC-0820) filed July 7,2005. The embodiment provided in Figure 18 further includes a flow meter 318 comprising an array of ultrasonic sensors similar to that shown and described in Figure 1, Figure 4 and Figure 16. The flow meter 318 may be used in combination with the passive flow meter 312 and differential pressure (DP) meter, or simply in combination with the DP meter.

One will appreciate that while each of the apparatus includes devices in a particular order on the pipe, one will appreciate that the device may be disposed in any order.

The dimensions and/or geometries for any of the embodiments described herein are merely for illustrative purposes and, as such, any other dimensions and/or geometries may be used if desired, depending on the application, size, performance, manufacturing requirements, or other factors, in view of the teachings herein.

It should be understood that, unless stated otherwise herein, any of the features characteristics, alternatives or modifications described regarding a particular embodiment herein may also be applied, used, or incorporated with any other embodiment described herein. Also, the drawings herein are not drawn to scale.

Although the invention has been described and illustrated with respect to exemplary embodiments thereof, the foregoing and various other additions and omissions may be made therein and thereto without departing from the scope of the present invention.

**Claims**

1. An apparatus for determining a characteristic of an aerated fluid (102) flowing within a pipe (104), the apparatus comprising:

   at least one first sensing device (112-118, 120-126) associated with the pipe to sense a low-frequency component and a high-frequency component of the aerated fluid flow, wherein said at least one first sensing device generates first sensor data responsive to said low-frequency component of the aerated fluid indicative of speed of sound at frequencies below a bubble resonant frequency being a transition frequency below which the speed of sound propagating through the fluid is influenced by entrained gases and above which the entrained gases have no significant influence, and second sensor data indicative of a volumetric flow rate responsive to said high-frequency component of the aerated fluid flow;
   at least one second sensing device (302) associated with the pipe to sense a density or mass flow of the aerated fluid flow and to generate third sensor data indicative of a density or a mass flow rate responsive to said density or mass flow measurement of the aerated fluid flow; and
   a processing device (108), wherein said processing device is communicated with said at least one first sensing device and said at least one second sensing device to receive and process said first sensor data, said second sensor data and said third sensor data to generate fluid data responsive to a characteristic of the aerated fluid flow.

2. The apparatus of claim 1, wherein said characteristic of the aerated fluid flow is at least one of a Gas Volume Fraction (GVF), a volumetric flow rate, a WaterCut value indicative of a phase fraction of water, a liquid flow rate and a net/oil/water rate.

3. The apparatus of claim 1, wherein said second sensor data is responsive to the density or mass flow measurement and the speed of sound through a liquid component of the fluid flow.

4. The apparatus of claim 1, wherein said at least one first sensing device includes a plurality of sensing devices (112-118, 120-126), wherein said plurality of sensing devices are axially distributed along the pipe.

5. The apparatus of claim 1, wherein said at least one first sensing device includes a transmitting device (TX) and a receiving device (RX), wherein when said transmitting device and said receiving device are associated with the pipe, said transmitting device and said receiving device are disposed on opposing sides of the pipe.

6. The apparatus of claim 1, wherein the apparatus includes a fastening device for removably and securely associating the apparatus with the pipe, wherein the fastening device is configured for easy removal and installation.

7. The apparatus of claim 1, wherein the apparatus is securely associated with an external portion of the pipe via a clamp-on device.

8. The apparatus of claim 1, wherein
   the aerated fluid is a three phase multiphase fluid;
   the at least one first sensing device, includes a flow sensing device (318, 312, 314) and a watercut sensing device (316) for sensing a watercut value indicative of a phase fraction of water;
   the at least one second sensing device includes a density sensing device (302);
   the processing device (108,320) including multiphase flow model logic (400), wherein said processing device (108,320) configured to receive flow rate data from said flow sensing device (312,318), density data from said density sensing device (302), and watercut data from said watercut sensing device (316),
   wherein the multiphase flow model logic (400) receives the data from each of the flow, density and watercut sensing devices and processes the data to optimize and correct for any errors, inaccuracies, and/or various flow conditions or regimes,
wherein the fluid data is three phase flow measurement data output by said multiphase flow model logic (400).

9. The apparatus of claim 8, wherein the flow sensing device, the density sensing device and the watercut sensing device are secured to the outer surface of the pipe.


**Patentansprüche**

1. Vorrichtung zur Bestimmung einer Charakteristik eines mit Luft durchsetzten Fluids (102), welches innerhalb einer Röhre (104) fließt, wobei die Vorrichtung aufweist:

wenigstens eine erste Erfassungsvorrichtung (112-118, 120-126), welche mit der Röhre assoziiert ist, um eine Niedrigfrequenzkomponente und eine Hochfrequenzkomponente des mit Luft durchsetzten Fluidstroms zu erfassen, wobei die wenigstens eine erste Erfassungsvorrichtung erste Sensordaten in Reaktion auf die Niedrigfrequenzkomponente des mit Luft durchsetzten Fluids generiert, die bezeichnend sind für Schallgeschwindigkeit an Frequenzen unterhalb einer Blasenresonanzfrequenz, welche eine Übergangsfrequenz ist, unterhalb welcher die Geschwindigkeit des Schalls, welcher sich durch das Fluid ausbreitet, durch eingeschlossene Gase beeinflusst wird, und oberhalb welcher die eingeschlossenen Gase keinen signifikanten Einfluss haben, und zweite Sensordaten, die für eine Volumenflussrate in Reaktion auf die Hochfrequenzkomponente des mit Luft durchsetzten Fluidstroms bezeichnet sind;
wenigstens eine zweite Erfassungsvorrichtung (302), welche mit der Röhre assoziiert ist, um eine Dichte oder einen Massenfluss des mit Luft durchsetzten Fluidstroms zu erfassen und um dritte Sensordaten zu generieren, die bezeichnend sind für eine Dichte oder eine Massenflussrate in Reaktion auf die Dichte- oder Massenflussmessung des mit Luft durchsetzten Fluidstroms; und
eine Verarbeitungsvorrichtung (108), wobei die Verarbeitungsvorrichtung kommunikativ mit der wenigstens einen ersten Erfassungsvorrichtung und der wenigstens einen zweiten Erfassungsvorrichtung verbunden ist, um die ersten Sensordaten, die zweiten Sensordaten und die dritten Sensordaten zu empfangen und zu verarbeiten, um Fluiddaten in Reaktion auf eine Charakteristik des mit Luft durchsetzten Fluidstroms zu generieren.

2. Vorrichtung nach Anspruch 1, wobei die Charakteristik des mit Luft durchsetzten Fluidstroms wenigstens eines aus einem Gasvolumenanteil (GVF), einer Volumenflussrate, eines Wasserabtragswerts, welcher bezeichnend ist für einen Phasenanteil von Wasser, einer Flüssigkeitsflussrate und eine Netto/Öl/Wasser-Rate ist.

3. Vorrichtung nach Anspruch 1, wobei die zweiten Sensordaten reagierend auf die Dichte- oder Massenflussmessung und die Geschwindigkeit des Schalls durch eine Flüssigkomponente des Fluidstroms sind.

4. Vorrichtung nach Anspruch 1, wobei die wenigstens eine erste Erfassungsvorrichtung eine Mehrzahl von Erfassungsvorrichtungen (112-118, 120-126) beinhaltet, wobei die Mehrzahl von Erfassungsvorrichtungen axial entlang der Röhre verteilt sind.

5. Vorrichtung nach Anspruch 1, wobei die wenigstens eine erste Erfassungsvorrichtung eine Übertragungsvorrichtung (TX) und eine Empfangsvorrichtung (RX) beinhaltet, wobei wenn die Übertragungsvorrichtung und die Empfangsvorrichtung mit der Röhre assoziiert sind, die Übertragungsvorrichtung und die Empfangsvorrichtung auf gegenüberliegenden Seiten der Röhre angeordnet sind.

6. Vorrichtung nach Anspruch 1, wobei die Vorrichtung eine Befestigungsvorrichtung für ein lösbares und sicheres Assoziieren der Vorrichtung mit der Röhre beinhaltet, wobei die Befestigungsvorrichtung für ein einfaches Lösen und einfache Installation konfiguriert ist.

7. Vorrichtung nach Anspruch 1, wobei die Vorrichtung in sicherer Weise mit einem externen Teil der Röhre mittels einer Klemmbefestigungsvorrichtung assoziiert ist.

8. Vorrichtung nach Anspruch 1, wobei
das mit Luft durchsetzte Fluid ein Dreiphasen-Mehrphasenfluid ist; die wenigstens eine erste Erfassungsvorrichtung eine Flusserfassungsvorrichtung (318; 312, 314) und eine Wasserabtrag-Erfassungsvorrichtung (316) zur Erfassung eines Wasserabtragswerts, welcher bezeichnend ist für einen Phasenanteil des Wassers, beinhaltet;
wobei die wenigstens eine zweite Erfassungsvorrichtung eine Dichteerfassungsvorrichtung (302) beinhaltet;

wobei die Verarbeitungsvorrichtung (108, 320) eine Multiphasen-Flussmodelllogik (400) beinhaltet, wobei die Verarbeitungsvorrichtung (108, 320) konfiguriert ist, Flussraten-Daten von der Flusserfassungsvorrichtung (312, 318), Dichtedaten von der Dichteerfassungsvorrichtung (302) und Wasserabtragsdaten von der Wasserabtragserfassungsvorrichung (316) zu empfangen,

wobei die Multiphasen-Flussmodelllogik (400) die Daten von jeder der Fluss-, Dichte- und Wasserabtragserfassungsvorrichtungen empfängt und die Daten verarbeitet, um irgendwelche Fehler, Ungenauigkeiten und/oder verschiedene Flussbedingungen oder -charakteristiken zu optimieren und korrigieren,

wobei die Fluiddaten Messdaten eines Dreiphasenflusses sind, welche durch die Multiphasen-Flussmodelllogik (400) ausgegeben werden.

9.  Vorrichtung nach Anspruch 8, wobei die Flusserfassungsvorrichtung, die Dichteerfassungsvorrichtung und die Wasserabtragserfassungsvorrichtung an der äußeren Oberfläche der Röhre angebracht sind.

**Revendications**

1.  Appareil pour déterminer une caractéristique d'un fluide aéré (102) s'écoulant au sein d'un tuyau (104), l'appareil comprenant :

    au moins un premier dispositif de détection (112-118, 120-126) associé au tuyau pour détecter une composante basse fréquence et une composante haute fréquence de l'écoulement de fluide aéré, ledit au moins un premier dispositif de détection générant des premières données de détection en réponse à ladite composante basse fréquence du fluide aéré indiquant la vitesse du son à des fréquences inférieures à la fréquence de résonance de bulle qui représente la fréquence de transition en dessous de laquelle la vitesse du son qui se propage à travers le fluide est influencée par des gaz entraînés et au-dessus de laquelle les gaz entraînés n'ont aucune influence significative, et des deuxièmes données de détection indiquant un débit volumique, en réponse à ladite composante haute fréquence de l'écoulement de fluide aéré ;
    au moins un deuxième dispositif de détection (302) associé au tuyau pour détecter une densité ou un débit massique de l'écoulement de fluide aéré et pour générer des troisièmes données de détection indiquant une densité ou un débit massique, en réponse à ladite mesure de densité ou du débit massique de l'écoulement de fluide aéré ; et
    un dispositif de traitement (108), ledit dispositif de traitement étant mis en communication avec ledit au moins un premier dispositif de détection et avec ledit au moins un deuxième dispositif de détection pour recevoir et traiter lesdites premières données de détection, lesdites deuxièmes données de détections et lesdites troisièmes données de détection afin de générer des données de fluide en réponse à une caractéristique de l'écoulement de fluide aéré.

2.  Appareil selon la revendication 1, dans lequel ladite caractéristique de l'écoulement de fluide aéré représente au moins une caractéristique choisie parmi le groupe comprenant la fraction de volume de gaz (GVF), le débit volumique, la valeur de proportion d'eau indiquant la fraction phasique de l'eau, le débit de liquide et le rapport du volume net d'huile/d'eau.

3.  Appareil selon la revendication 1, dans lequel lesdites deuxièmes données de détection sont sensibles à la mesure de la densité ou du débit et à la vitesse du son à travers une composante liquide de l'écoulement de fluide.

4.  Appareil selon la revendication 1, dans lequel ledit au moins un premier dispositif de détection englobe plusieurs dispositifs de détection (112-118, 120-126), lesdits plusieurs dispositifs de détection étant distribués en direction axiale le long du tuyau.

5.  Appareil selon la revendication 1, dans lequel ledit au moins un premier dispositif de détection englobe un dispositif d'émission (TX) et un dispositif de réception (RX), ledit dispositif d'émission et ledit dispositif de réception étant associés au tuyau, ledit dispositif d'émission et ledit dispositif de réception étant disposés sur les côtés opposés du tuyau.

6.  Appareil selon la revendication 1, dans lequel l'appareil englobe un dispositif de fixation pour associer de manière amovible et de manière résistante l'appareil au tuyau, le dispositif de fixation étant configuré pour un montage et un retrait rapides.

**7.** Appareil selon la revendication 1, dans lequel l'appareil est associé de manière résistante à une portion externe du tuyau via un dispositif de serrage.

**8.** Appareil selon la revendication 1, dans lequel :

le fluide aéré est un fluide polyphasique à trois phases ;
ledit au moins un premier dispositif de détection englobe un dispositif de détection de l'écoulement (318, 312, 314) et un dispositif de détection de la proportion d'eau indiquant la fraction phasique de l'eau ;
ledit au moins un deuxième dispositif de détection englobe un dispositif de détection de la densité (302) ;
le dispositif de traitement (108, 320) englobe une logique de modèle d'écoulement polyphasique (400), ledit dispositif de traitement (108, 320) étant configuré pour recevoir des données de débit à partir dudit dispositif de détection de l'écoulement (312, 318), des données de densité à partir dudit dispositif de détection de la densité (302) et des données de la proportion d'eau à partir dudit dispositif de détection de la proportion d'eau (316) ;
dans lequel la logique de modèle d'écoulement polyphasique (400) reçoit les données émises par chacun des dispositifs de détection de l'écoulement, de la densité et de la proportion d'eau et traite les données pour optimiser et pour corriger n'importe quelle erreur, imprécision et/ou diverses conditions ou divers régimes d'écoulement ;
dans lequel les données de fluide représentent des données de mesure d'écoulement triphasique fournies par ladite logique de modèle d'écoulement polyphasique (400).

**9.** Appareil selon la revendication 8, dans lequel le dispositif de détection de l'écoulement, le dispositif de détection de la densité et le dispositif de détection de la proportion d'eau sont fixés à la surface externe du tuyau.

EP 1 982 169 B1

100

108

GVF Logic

SOS$_{Mix}$

158

SOS$_{MIX}$ Logic

138

$P_1(t) - P_N(t)$

170

Flow Logic

Watercut Logic

SOS$_{Liq}$

186

SOS$_{Liq}$ Logic

→ GVF

→ SOS$_{MIX}$

→ Flow Velocity

→ Volumetric Flow Rate

→ SOS$_{LIQ}$

→ Water Cut

→ Composition

102

104

102

Gas Volume Fraction Meter

U/S Flow Meter

U/S Watercut Meter

**Figure 1**

Figure 2.

EP 1 982 169 B1

Figure 3

**Figure 4**

**Figure 5**

EP 1 982 169 B1

```
┌─────────────────────┐
│  Device for Measuring│
│  Speed of Sound (SOS)│─────────────────┐
│    Within a Pipe     │                 │
└─────────────────────┘                 │
                                        ▼
┌─────────────────────┐        ┌─────────────────────┐
│   Pressure* &/or     │        │    Entrained Air     │      Gas Volume
│   Temperature*       │───────▶│     Processing       │────▶  Fraction
│     Sensor           │        │       Unit           │
└─────────────────────┘        └─────────────────────┘
```

\* Measured or Estimated

**Figure 6**

```
┌─────────┐           ┌──────────────────────────────────┐
│         │           │ ┌──────────────────────────────┐ │
│ P*, T*  │══════════▶│ │      Fluid Properties        │ │
│         │           │ │  * SOS, ρ for Water & Gas    │ │
└─────────┘           │ └──────────────────────────────┘ │
                      │              ║                    │
                      │              ▼                    │
┌─────────┐           │ ┌──────────────────────────────┐ │        ┌─────────┐
│         │           │ │      Entrained Gas           │ │        │         │
│  SOS    │══════════▶│ │    Volume Fraction           │═════════▶│   GVF   │
│         │           │ └──────────────────────────────┘ │        │         │
└─────────┘           └──────────────────────────────────┘        └─────────┘
```

**Figure 7** +

EP 1 982 169 B1

$T_1(t)$ $T_2(t)$ $T_3(t)$ $T_N(t)$
and/or
$P_1(t)$ $P_2(t)$ $P_3(t)$ $P_N(t)$

170

| Data Acquisition | 172 |

| FFT | 174 |

$P_1(\omega)$ $P_2(\omega)$ $P_3(\omega)$ $P_N(\omega)$

| Data Accumulator | 176 |

Array Processor

$\omega$ — Convective Ridge
k

178

| Convective Ridge Identifier | 182 |

| Analyzer | 184 |

Flow Rate     Vol. Flow Rate

**Figure 11**

---

$P_1(t)$ $P_2(t)$ $P_3(t)$ $P_N(t)$

138

| Data Acquisition | 140 |

| FFT | 142 |

$P_1(\omega)$ $P_2(\omega)$ $P_3(\omega)$ $P_N(\omega)$

| Data Accumulator | 144 |

Array Processor
Acoustic Ridges

$\omega$
k

146

| Acoustic Ridge Identifier | 154 |

| Analyzer | 156 |

$SOS_{Mixture}$

**Figure 8**

24

Figure 9

EP 1 982 169 B1

SOS as Function of Entrained Air

Figure 10

Figure 12

Convection Velocity
determined by the slope of
Convective Ridge
U=ω/k

Wave Number (k)

14 ft/s

Velocity (ft/s)

Optimization Function

Frequency (ω)

180

# Ultrasonic SOS = SOS$_{liq}$

## Example: Oil & Water Mixture

$$\phi_{water} = f\left(SOS_{liq}, SOS_{oil}, SOS_{water}, \rho_{oil}, \rho_{water}\right)$$

<u>Figure 13</u>

EP 1 982 169 B1

**Figure 14**

Figure 15.

GVF

SOS$_{MIX}$

Flow Velocity

Volumetric Flow
Rate

SOS$_{LIQ}$

Water Cut

Composition

Liquid Flow Rate

Net/Oil/Water Rate

108

300

158

GVF Logic

Watercut Logic

SOS$_{Mix}$

138

186

SOS$_{MIX}$ Logic

SOS$_{Liq}$ Logic

SOS$_{Liq}$

GVF

$P_1(t) - P_N(t)$

170

Correction
Logic

Flow Logic

302

314

318

316

104

102

Density/
Mass Flow
Meter

Gas
Volume
Fraction
Meter

U/S
Flow
Meter

U/S
Watercut
Meter

**Figure 16**

Figure 17

Figure 18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11442954 B
- US 68553205 P
- US 73668405 P
- US 75824206 P
- US 20040168522 A1
- US 4491008 A
- WO 0169040 A1
- US 5083452 A
- US 2005193832 A
- US 349716 A
- US 376427 A
- US 76241004 A
- US 34409499 A
- US 6354147 B
- US 79511104 A
- US 99722101 A
- US 6587798 B
- US 007749 A
- US 75697704 A
- US 729994 A
- US 6609069 B
- US 2874568 A
- US 4004461 A
- US 6532827 B
- US 4195517 A
- US 5856622 A
- US 6397683 B
- US 00774901 A
- US 00773601 A
- US 35978502 P
- US 42543602 P
- US 72999400 A
- US 10875857 A
- US 71281803 A
- US 92559897 A
- US 6016702 A
- US 224821 A
- US 10892886 B
- US 60724952 B
- US 60697479 B